# EUROPEAN PATENT APPLICATION

(11) **EP 4 407 046 A2**
(43) Date of publication of application: **31.07.2024**
(21) Application number: 24172440.0
(22) Date of filing: 20.03.2020
(51) Int. Cl.: C12Q 1/689

(54) **COMPOSITIONS AND METHODS FOR DETECTING GROUP A STREPTOCOCCUS**

(30) Priority: 22.03.2019 US 201962822678 P
(62) Divisional of application: 20718492.0
(71) Applicant: Gen-Probe Incorporated, San Diego, CA 92121 (US)
(72) Inventor: PANUGANTI, Sree Divya, San Diego, 92121 (US); SHAH, Ankur, San Diego, 92121 (US)
(74) Representative: Script IP Limited

(57) **Abstract**

There is described a composition or kit comprising at least first and second amplification oligomers, wherein:(a) the first amplification oligomer and second amplification oligomer are configured to amplify a Group A Streptococcus amplicon; (b) the first amplification oligomer: (i) comprises a first sequence which is SEQ ID NO: 4; (ii) comprises a sequence with 0, 1, or 2 mismatches to a first sequence which is SEQ ID NO: 4; (iii) competes for hybridization to a Group A Streptococcus (GAS) nucleic acid under stringent conditions with an amplification oligomer whose sequence consists of a first sequence which is SEQ ID NO: 4; or (iv) comprises at least 10, 11, 12, 13, 14, 15, 16, 17, 18, 19, or 20 contiguous nucleotides of a first sequence which is SEQ ID NO: 4; and (c) the second amplification oligomer: (i) comprises a second sequence which is SEQ ID NO: 6; (ii) comprises a sequence with 0, 1, or 2 mismatches to a second sequence which is SEQ ID NO: 6; (iii) competes for hybridization to a Group A Streptococcus (GAS) nucleic acid under stringent conditions with an amplification oligomer whose sequence consists of a second sequence which is SEQ ID NO: 6; or (iv) comprises at least 10, 11, 12, 13, 14, 15, 16, 17, 18, 19, or 20 contiguous nucleotides of a second sequence which is SEQ ID NO: 6.

## Description

This application claims the benefit of priority of US Provisional Patent Application No. 62/711,678, filed March 22, 2019, which is incorporated herein by reference in its entirety.

The embodiments herein are directed to the field of detecting infectious agents, more specifically by using compositions and methods to detect Group A Streptococcus (GAS) bacteria and related sequences.

The instant application contains a Sequence Listing which has been submitted electronically in ASCII format and is hereby incorporated by reference in its entirety. Said ASCII copy, created on March 16, 2020, is named 2020-03-20_01159-0038-00PCT_Seq_Listing_ST25.txt and is 8.0 KB in size.

*Streptococcus pyogenes*, more commonly known as group A β-hemolytic Streptococcus, is the etiologic agent of a number of infections in humans including acute pharyngitis, sinusitis, lymphadenitis, pyoderma, endocarditis, meningitis, septicemia, tonsillitis, impetigo, and upper respiratory tract infections. *Streptococcus pyogenes* infections are of particular concern because serious complications such as glomerulonephritis, rheumatic fever and scarlet fever may result if left untreated. Group A β-hemolytic streptococci are universally susceptible to penicillin G, a fact that makes antimicrobial susceptibility testing for this organism unnecessary unless the patient is allergic to penicillin.

Over ninety percent of all streptococcal infections are caused by *Streptococcus pyogenes.* Asymptomatic carriers colonized in the nasopharynx, skin, vagina or rectum are thought to transmit this organism through close person-to-person contact. Contaminated food may also be a source of transmission and infections in humans.

Presumptive identification of *Streptococcus pyogenes* was traditionally based upon physiological and biochemical traits. These include colony morphology, β-hemolytic activity on sheep blood agar, gram strain, susceptibility to bacitracin, and the ability to hydrolyze L-pyrrolidonyl-β-naphthylamide (PYR). Commercial antibody tests such as latex agglutination targeted the Streptococcus group A antigen. Occasionally, these tests were shown to react positively with some strains of *Streptococcus anginosus* containing the group A antigen. In addition, these tests occasionally required repeat testing due to equivocal results. Serological grouping was the method of choice for definitive identification of *Streptococcus pyogenes.* Lancefield serological grouping is determined from group-specific carbohydrate antigen extracted from cell walls and group-specific antisera. This method can be time-consuming and costly, therefore most laboratories relied on the traditional physiological and biochemical methods.

More recently, nucleic acid assays have aided in the diagnosis of Group A Streptococcal pharyngitis. These assays use nucleic acid hybridization and/or amplification for the qualitative detection of Group A Streptococcal DNA and RNA. Such tests offer a non-subjective, accurate and rapid identification method for definitively identifying *Streptococcus pyogenes* from throat swabs. Identification is based, e.g., upon the detection of specific ribosomal RNA sequences that are unique to *Streptococcus pyogenes.*

Nonetheless, due to factors such as interstrain variation and similarity of Streptococcus species other than GAS that may give false positives with existing reagents, there remains a need for compositions and methods that provide even more sensitive and specific detection and quantification of GAS. This disclosure aims to meet these needs, provide other benefits, or at least provide the public with a useful choice.

Accordingly, the following embodiments are provided. Embodiment 1 is a composition or kit comprising at least first and second amplification oligomers, wherein the first and second amplification oligomers comprise first and second sequences, respectively, and the first and second sequences are the sequences of one of the following pairs of SEQ ID NOs, respectively: 1 and 3; 4 and 6; 7 and 9; 10 and 12; 13 and 15; 16 and 17; 19 and 20; 22 and 23; 27 and 28; 30 and 28; 31 and 32; 34 and 35; or 37 and 38.

Embodiment 2 is a composition or kit comprising at least first and second amplification oligomers, wherein:
(a) the first amplification oligomer and second amplification oligomer are configured to amplify a Group A Streptococcus amplicon;
(b) the first amplification oligomer:
   (i) comprises a first sequence which is SEQ ID NO: 1, 4, 7, 10, 13, 16, 19, 22, 27, 30, 31, 34, or 37;
   (ii) comprises a sequence with 0, 1, or 2 mismatches to a first sequence which is SEQ ID NO: 1, 4, 7, 10, 13, 16, 19, 22, 27, 30, 31, 34, or 37;
   (iii) competes for hybridization to a Group A Streptococcus (GAS) nucleic acid under stringent conditions with an amplification oligomer whose sequence consists of a first sequence which is SEQ ID NO: 1, 4, 7, 10, 13, 16, 19, 22, 27, 30, 31, 34, or 37; or
   (iv) comprises at least 10, 11, 12, 13, 14, 15, 16, 17, 18, 19, or 20 contiguous nucleotides of a first sequence which is SEQ ID NO: 1, 4, 7, 10, 13, 16, 19, 22, 27, 30, 31, 34, or 37; and
(c) the second amplification oligomer:
   (i) comprises a second sequence which is SEQ ID NO: 3, 6, 9, 12, 15, 17, 20, 23, 28, 32, 35, or 38;
   (ii) comprises a sequence with 0, 1, or 2 mismatches to a second sequence which is SEQ ID NO: 3, 6, 9, 12, 15, 17, 20, 23, 28, 32, 35, or 38;
   (iii) competes for hybridization to a Group A Streptococcus (GAS) nucleic acid under stringent conditions with an amplification oligomer whose sequence consists of a second sequence which is SEQ ID NO: 3, 6, 9, 12, 15, 17, 20, 23, 28, 32, 35, or 38; or
   (iv) comprises at least 10, 11, 12, 13, 14, 15, 16, 17, 18, 19, or 20 contiguous nucleotides of a second sequence which is SEQ ID NO: 3, 6, 9, 12, 15, 17, 20, 23, 28, 32, 35, or 38.

Embodiment 3 is a method of detecting GAS in a sample, the method comprising:
contacting the sample with at least first and second amplification oligomers, thereby forming a composition,
performing a nucleic acid amplification reaction in the composition which produces a GAS amplicon in the presence of a GAS nucleic acid, and
detecting the presence or absence of the at least one amplicon,
wherein (a) the first amplification oligomer:
   (i) comprises a first sequence which is SEQ ID NO: 1, 4, 7, 10, 13, 16, 19, 22, 27, 30, 31, 34, or 37;
   (ii) comprises a sequence with 0, 1, or 2 mismatches to a first sequence which is SEQ ID NO: 1, 4, 7, 10, 13, 16, 19, 22, 27, 30, 31, 34, or 37;
   (iii) competes for hybridization to a Group A Streptococcus (GAS) nucleic acid under stringent conditions with an amplification oligomer whose sequence consists of a first sequence which is SEQ ID NO: 1, 4, 7, 10, 13, 16, 19, 22, 27, 30, 31, 34, or 37; or
   (iv) comprises at least 10, 11, 12, 13, 14, 15, 16, 17, 18, 19, or 20 contiguous nucleotides of a first sequence which is SEQ ID NO: 1, 4, 7, 10, 13, 16, 19, 22, 27, 30, 31, 34, or 37; and
(b) the second amplification oligomer:
   (i) comprises a second sequence which is SEQ ID NO: 3, 6, 9, 12, 15, 17, 20, 23, 28, 32, 35, or 38;
   (ii) comprises a sequence with 0, 1, or 2 mismatches to a second sequence which is SEQ ID NO: 3, 6, 9, 12, 15, 17, 20, 23, 28, 32, 35, or 38;
   (iii) competes for hybridization to a Group A Streptococcus (GAS) nucleic acid under stringent conditions with an amplification oligomer whose sequence consists of a second sequence which is SEQ ID NO: 3, 6, 9, 12, 15, 17, 20, 23, 28, 32, 35, or 38; or
   (iv) comprises at least 10, 11, 12, 13, 14, 15, 16, 17, 18, 19, or 20 contiguous nucleotides of a second sequence which is SEQ ID NO: 3, 6, 9, 12, 15, 17, 20, 23, 28, 32, 35, or 38.

Embodiment 4 is the composition, kit, or method of any one of the preceding embodiments, wherein the first amplification oligomer comprises a sequence with 0, 1, or 2 mismatches to a first sequence which is SEQ ID NO: 1, 4, 7, 10, 13, 16, 19, 22, 27, 30, 31, 34, or 37.

Embodiment 5 is the composition, kit, or method of any one of the preceding embodiments, wherein the first amplification oligomer competes for hybridization to a Group A Streptococcus (GAS) nucleic acid under stringent conditions with an amplification oligomer whose sequence consists of a first sequence which is SEQ ID NO: 1, 4, 7, 10, 13, 16, 19, 22, 27, 30, 31, 34, or 37.

Embodiment 6 is the composition, kit, or method of any one of the preceding embodiments, wherein the first amplification oligomer comprises at least 10, 11, 12, 13, 14, 15, 16, 17, 18, 19, or 20 contiguous nucleotides of a first sequence which is SEQ ID NO: 1, 4, 7, 10, 13, 16, 19, 22, 27, 30, 31, 34, or 37.

Embodiment 7 is the composition, kit, or method of any one of the preceding embodiments, wherein the first amplification oligomer comprises a first sequence which is SEQ ID NO: 1, 4, 7, 10, 13, 16, 19, 22, 27, 30, 31, 34, or 37.

Embodiment 8 is the composition, kit, or method of any one of the preceding embodiments, wherein the second amplification oligomer comprises a sequence with 0, 1, or 2 mismatches to a second sequence which is SEQ ID NO: 3, 6, 9, 12, 15, 17, 20, 23, 28, 32, 35, or 38.

Embodiment 9 is the composition, kit, or method of any one of the preceding embodiments, wherein the second amplification oligomer competes for hybridization to a Group A Streptococcus (GAS) nucleic acid under stringent conditions with an amplification oligomer whose sequence consists of a second sequence which is SEQ ID NO: 3, 6, 9, 12, 15, 17, 20, 23, 28, 32, 35, or 38.

Embodiment 10 is the composition, kit, or method of any one of the preceding embodiments, wherein the second amplification oligomer comprises at least 10, 11, 12, 13, 14, 15, 16, 17, 18, 19, or 20 contiguous nucleotides of a second sequence which is SEQ ID NO: 3, 6, 9, 12, 15, 17, 20, 23, 28, 32, 35, or 38.

Embodiment 11 is the composition, kit, or method of any one of the preceding embodiments, wherein the second amplification oligomer comprises a second sequence which is SEQ ID NO: 3, 6, 9, 12, 15, 17, 20, 23, 28, 32, 35, or 38.

Embodiment 12 is the composition, kit, or method of any one of embodiments 1-11, wherein the first sequence is the sequence of SEQ ID NO: 1.

Embodiment 13 is the composition, kit, or method of any one of embodiments 1-11, wherein the first sequence is the sequence of SEQ ID NO: 4.

Embodiment 14 is the composition, kit, or method of any one of embodiments 1-11, wherein the first sequence is the sequence of SEQ ID NO: 7.

Embodiment 15 is the composition, kit, or method of any one of embodiments 1-11, wherein the first sequence is the sequence of SEQ ID NO: 10.

Embodiment 16 is the composition, kit, or method of any one of embodiments 1-11, wherein the first sequence is the sequence of SEQ ID NO: 13.

Embodiment 17 is the composition, kit, or method of any one of embodiments 1-11, wherein the first sequence is the sequence of SEQ ID NO: 16.

Embodiment 18 is the composition, kit, or method of any one of embodiments 1-11, wherein the first sequence is the sequence of SEQ ID NO: 19.

Embodiment 19 is the composition, kit, or method of any one of embodiments 1-11, wherein the first sequence is the sequence of SEQ ID NO: 22.

Embodiment 20 is the composition, kit, or method of any one of embodiments 1-11, wherein the first sequence is the sequence of SEQ ID NO: 27.

Embodiment 21 is the composition, kit, or method of any one of embodiments 1-11, wherein the first sequence is the sequence of SEQ ID NO: 30.

Embodiment 22 is the composition, kit, or method of any one of embodiments 1-11, wherein the first sequence is the sequence of SEQ ID NO: 31.

Embodiment 23 is the composition, kit, or method of any one of embodiments 1-11, wherein the first sequence is the sequence of SEQ ID NO: 34.

Embodiment 24 is the composition, kit, or method of any one of embodiments 1-11, wherein the first sequence is the sequence of SEQ ID NO: 37.

Embodiment 25 is the composition, kit, or method of any one embodiments 1-24, wherein the second sequence is the sequence of SEQ ID NO: 3.

Embodiment 26 is the composition, kit, or method of any one embodiments 1-24, wherein the second sequence is the sequence of SEQ ID NO: 6.

Embodiment 27 is the composition, kit, or method of any one embodiments 1-24, wherein the second sequence is the sequence of SEQ ID NO: 9.

Embodiment 28 is the composition, kit, or method of any one embodiments 1-24, wherein the second sequence is the sequence of SEQ ID NO: 12.

Embodiment 29 is the composition, kit, or method of any one embodiments 1-24, wherein the second sequence is the sequence of SEQ ID NO: 15.

Embodiment 30 is the composition, kit, or method of any one embodiments 1-24, wherein the second sequence is the sequence of SEQ ID NO: 17.

Embodiment 31 is the composition, kit, or method of any one embodiments 1-24, wherein the second sequence is the sequence of SEQ ID NO: 20.

Embodiment 32 is the composition, kit, or method of any one embodiments 1-24, wherein the second sequence is the sequence of SEQ ID NO: 23.

Embodiment 33 is the composition, kit, or method of any one embodiments 1-24, wherein the second sequence is the sequence of SEQ ID NO: 28.

Embodiment 34 is the composition, kit, or method of any one embodiments 1-24, wherein the second sequence is the sequence of SEQ ID NO: 32.

Embodiment 35 is the composition, kit, or method of any one embodiments 1-24, wherein the second sequence is the sequence of SEQ ID NO: 35.

Embodiment 36 is the composition, kit, or method of any one embodiments 1-24, wherein the second sequence is the sequence of SEQ ID NO: 38.

Embodiment 37 is the composition, kit, or method of any one of the preceding embodiments, wherein the composition or kit further comprises a third oligomer, and the third oligomer:
(i) comprises a third sequence which is SEQ ID NO: 2, 5, 8, 11, 14, 18, 25, 26, 21, 24, 29, 33, 36, or 39;
(ii) comprises a sequence with 0, 1, or 2 mismatches to a third sequence which is SEQ ID NO: 2, 5, 8, 11, 14, 18, 25, 26, 21, 24, 29, 33, 36, or 39;
(iii) competes for hybridization to a Group A Streptococcus (GAS) nucleic acid under stringent conditions with an amplification oligomer whose sequence consists of a third sequence which is SEQ ID NO: 2, 5, 8, 11, 14, 18, 25, 26, 21, 24, 29, 33, 36, or 39; or
(iv) comprises at least 10, 11, 12, 13, 14, 15, 16, 17, 18, 19, or 20 contiguous nucleotides of a third sequence which is SEQ ID NO: 2, 5, 8, 11, 14, 18, 25, 26, 21, 24, 29, 33, 36, or 39.

Embodiment 38 is the composition, kit, or method of embodiment 37, wherein the third oligomer comprises a sequence with 0, 1, or 2 mismatches to a third sequence which is SEQ ID NO: 2, 5, 8, 11, 14, 18, 25, 26, 21, 24, 29, 33, 36, or 39.

Embodiment 39 is the composition, kit, or method of embodiment 37 or 38, wherein the third oligomer competes for hybridization to a Group A Streptococcus (GAS) nucleic acid under stringent conditions with an amplification oligomer whose sequence consists of a third sequence which is SEQ ID NO: 2, 5, 8, 11, 14, 18, 25, 26, 21, 24, 29, 33, 36, or 39.

Embodiment 40 is the composition, kit, or method of any one of embodiments 37-39, wherein the third oligomer comprises at least 10, 11, 12, 13, 14, 15, 16, 17, 18, 19, or 20 contiguous nucleotides of a third sequence which is SEQ ID NO: 2, 5, 8, 11, 14, 18, 25, 26, 21, 24, 29, 33, 36, or 39.

Embodiment 41 is the composition, kit, or method of embodiment 37, wherein the third oligomer comprises a third sequence which is SEQ ID NO: 2, 5, 8, 11, 14, 18, 25, 26, 21, 24, 29, 33, 36, or 39.

Embodiment 42 is the composition, kit, or method of embodiment any one of embodiments 37-41, wherein the third sequence is the sequence of SEQ ID NO: 2.

Embodiment 43 is the composition, kit, or method of any one of embodiments 37-41, wherein the third sequence is the sequence of SEQ ID NO: 5.

Embodiment 44 is the composition, kit, or method of any one of embodiments 37-41, wherein the third sequence is the sequence of SEQ ID NO: 8, optionally wherein the N in SEQ ID NO: 8 is an inosine.

Embodiment 45 is the composition, kit, or method of any one of embodiments 37-41, wherein the third sequence is the sequence of SEQ ID NO: 11, optionally wherein the N in SEQ ID NO: 11 is an inosine.

Embodiment 46 is the composition, kit, or method of any one of embodiments 37-41, wherein the third sequence is the sequence of SEQ ID NO: 14.

Embodiment 47 is the composition, kit, or method of any one of embodiments 37-41, wherein the third sequence is the sequence of SEQ ID NO: 18.

Embodiment 48 is the composition, kit, or method of any one of embodiments 37-41, wherein the third sequence is the sequence of SEQ ID NO: 21.

Embodiment 49 is the composition, kit, or method of any one of embodiments 37-41, wherein the third sequence is the sequence of SEQ ID NO: 24.

Embodiment 50 is the composition, kit, or method of any one of embodiments 37-41, wherein the third sequence is the sequence of SEQ ID NO: 25.

Embodiment 51 is the composition, kit, or method of any one of embodiments 37-41, wherein the third sequence is the sequence of SEQ ID NO: 26.

Embodiment 52 is the composition, kit, or method of any one of embodiments 37-41, wherein the third sequence is the sequence of SEQ ID NO: 29.

Embodiment 53 is the composition, kit, or method of any one of embodiments 37-41, wherein the third sequence is the sequence of SEQ ID NO: 33.

Embodiment 54 is the composition, kit, or method of any one of embodiments 37-41, wherein the third sequence is the sequence of SEQ ID NO: 36.

Embodiment 55 is the composition, kit, or method of any one of embodiments 37-41, wherein the third sequence is the sequence of SEQ ID NO: 39.

Embodiment 56 is the composition, kit, or method of any one of embodiments 37-55, wherein the third oligomer comprises a detectable label.

Embodiment 57 is the composition, kit, or method of embodiment 56, wherein the detectable label is a non-nucleotide detectable label.

Embodiment 58 is the composition, kit, or method of any one of embodiments 56-57, wherein the detectable label is fluorescent.

Embodiment 59 is the composition, kit, or method of embodiment 58, wherein the third oligomer comprising a fluorescent label further comprises a quencher.

Embodiment 60 is the composition, kit, or method of embodiment 59, wherein the quencher is positioned sufficiently proximal to the fluorescent label to quench at least 50%, 60%, 70%, 80%, 90%, 95%, 98%, or 99% of the fluorescence of the fluorescent label.

Embodiment 61 is the composition, kit, or method of embodiment 58-60, wherein at least one oligomer comprising a fluorescent label is a non-extendable oligomer.

Embodiment 62 is the composition, kit, or method of any one of the preceding embodiments, wherein at least one oligomer comprises one or more 5-methyl-dC residues.

Embodiment 63 is the composition, kit, or method of embodiment 62, wherein the one or more 5-methyl-dC residues comprise residues corresponding to one or more, or all, of positions 8, 18, and 24 of SEQ ID NO: 4.

Embodiment 64 is the composition, kit, or method of embodiment 62 or 63, wherein the one or more 5-methyl-dC residues comprise residues corresponding to one or more, or all, of positions 2, 4, 15, 19, and 21 of SEQ ID NO: 5.

Embodiment 65 is the composition, kit, or method of any one of embodiments 62-64, wherein the one or more 5-methyl-dC residues comprise residues corresponding to one or more, or all, of positions 7, 8, 11, and 13 of SEQ ID NO: 6.

Embodiment 66 is the composition, kit, or method of any one of embodiments 62-65, wherein the one or more 5-methyl-dC residues comprise residues corresponding to one or more, or all, of positions 7, 8, 10, and 21 of SEQ ID NO: 16.

Embodiment 67 is the composition, kit, or method of any one of embodiments 62-66, wherein the one or more 5-methyl-dC residues comprise residues corresponding to one or more, or all, of positions 6, 7, 9, 11, 12, 15, and 19 of SEQ ID NO: 17.

Embodiment 68 is the composition, kit, or method of any one of embodiments 62-67, wherein the one or more 5-methyl-dC residues comprise residues corresponding to one or more, or all, of positions 17 and 23 of SEQ ID NO: 18.

Embodiment 69 is the composition, kit, or method of any one of embodiments 62-68, wherein the one or more 5-methyl-dC residues comprise residues corresponding to one or more, or all, of positions 5, 7, 11, 13, and 19 of SEQ ID NO: 23.

Embodiment 70 is the composition, kit, or method of any one of embodiments 62-69, wherein the one or more 5-methyl-dC residues comprise residues corresponding to one or more, or all, of positions 17, 23, 25, 26, and 28 of SEQ ID NO: 25.

Embodiment 71 is the composition, kit, or method of any one of embodiments 62-70, wherein the one or more 5-methyl-dC residues comprise residues corresponding to one or more, or all, of positions 8, 14, 15, 16, 17, and 28 of SEQ ID NO: 26.

Embodiment 72 is the method of any one of embodiments 3-71, wherein the presence or absence of the amplicon is detected according to the occurrence or non-occurrence of hybridization of the third oligomer to the amplicon.

Embodiment 73 is the method of any one of embodiments 3-72 wherein the nucleic acid amplification reaction comprises thermal cycling.

Embodiment 74 is the method of any one of embodiment 3-73, wherein the nucleic acid amplification reaction comprises PCR.

Embodiment 75 is the method of any one of embodiments 3-74, wherein the nucleic acid amplification reaction comprises PCR with a polymerase with 5'-to-3' exonuclease activity, and detecting an amplicon using a probe oligomer comprising a fluorophore and a quencher, wherein exonucleolysis of the probe by the polymerase reduces quenching of fluoresence by the quencher.

Embodiment 76 is the composition, kit, or method of any one of the preceding embodiments, wherein the GAS amplicon is about 100-200 nucleotides in length.

Embodiment 77 is the composition, kit, or method of any one of the preceding embodiments, wherein the GAS amplicon is about 90-150 nucleotides in length.

Embodiment 78 is a kit according to any one of embodiments 1-2, 4-71, 76, or 77.

Embodiment 79 is a composition according to any one of embodiments 1-2, 4-71, 76, or 77.

Embodiment 80 is the composition of embodiment 79, which is aqueous, frozen, or lyophilized.

Embodiment 81 is a use of a composition or kit of any one of embodiments 78-80 for detecting or quantifying a GAS nucleic acid in a sample.

Embodiment 82 is a method of making an amplification oligonucleotide recited in any one of embodiments 1-36 comprising the step of chemically synthesizing the amplification oligonucleotide using sequential solid phase oligonucleotide synthesis.

Embodiment 83 is a method of making an oligomer of any one of embodiments 37-71, the method comprising chemically synthesizing the oligomer using sequential solid phase oligonucleotide synthesis.

### DETAILED DESCRIPTION OF CERTAIN EMBODIMENTS

### Definitions

Before describing the present teachings in detail, it is to be understood that the disclosure is not limited to specific compositions or process steps, as such may vary. It should be noted that, as used in this specification and the appended claims, the singular form "a," "an," and "the" include plural references unless the context clearly dictates otherwise. Thus, for example, reference to "an oligomer" includes a plurality of oligomers and the like. The conjunction "or" is to be interpreted in the inclusive sense, i.e., as equivalent to "and/or," unless the inclusive sense would be unreasonable in the context.

It will be appreciated that there is an implied "about" prior to the temperatures, concentrations, times, etc. discussed in the present disclosure, such that slight and insubstantial deviations are within the scope of the present teachings herein. In general, the term "about" indicates insubstantial variation in a quantity of a component of a composition not having any significant effect on the activity or stability of the composition. All ranges are to be interpreted as encompassing the endpoints in the absence of express exclusions such as "not including the endpoints"; thus, for example, "within 10-15" includes the values 10 and 15. Also, the use of "comprise," "comprises," "comprising," "contain," "contains," "containing," "include," "includes," and "including" are not intended to be limiting. It is to be understood that both the foregoing general description and detailed description are exemplary and explanatory only and are not restrictive of the teachings. To the extent that any material incorporated by reference is inconsistent with the express content of this disclosure, the express content controls.

Unless specifically noted, embodiments in the specification that recite "comprising" various components are also contemplated as "consisting of" or "consisting essentially of" the recited components; embodiments in the specification that recite "consisting of" various components are also contemplated as "comprising" or "consisting essentially of" the recited components; and embodiments in the specification that recite "consisting essentially of" various components are also contemplated as "consisting of" or "comprising" the recited components (this interchangeability does not apply to the use of these terms in the claims). "Consisting essentially of" means that additional component(s), composition(s) or method step(s) that do not materially change the basic and novel characteristics of the compositions and methods described herein may be included in those compositions or methods. Such characteristics include the ability to detect a GAS nucleic acid sequence present in a sample with specificity that distinguishes the GAS nucleic acid from other known Streptococci, optionally at a sensitivity that can detect about 50 CFU/mL or less of GAS (e.g., 10, 5, or 4 CFU/mL), and, optionally within about 2.5 hours or within about 45 cycles from the beginning of an amplification reaction when a cycled amplification reaction is used.

A "sample" or "specimen," including "biological" or "clinical" samples, refers to a tissue or material derived from a living or dead human or animal which may contain a GAS target nucleic acid, including, for example, nasopharyngeal or throat swabs, nasal or bronchial or broncheoaveolar washes, nasal aspirates, sputum, other respiratory tissue or exudates, biopsy tissue including lymph nodes, or body fluids such as blood or urine. A sample may be treated to physically or mechanically disrupt tissue or cell structure to release intracellular nucleic acids into a solution which may contain enzymes, buffers, salts, detergents and the like, to prepare the sample for analysis.

"Nucleic acid" and "polynucleotide" refer to a multimeric compound comprising nucleosides or nucleoside analogs which have nitrogenous heterocyclic bases or base analogs linked together to form a polynucleotide, including conventional RNA, DNA, mixed RNA-DNA, and polymers that are analogs thereof. A nucleic acid "backbone" may be made up of a variety of linkages, including one or more of sugar-phosphodiester linkages, peptide-nucleic acid bonds ("peptide nucleic acids" or PNA; PCT No. WO 95/32305), phosphorothioate linkages, methylphosphonate linkages, or combinations thereof. Sugar moieties of a nucleic acid may be ribose, deoxyribose, or similar compounds with substitutions, e.g., 2' methoxy or 2' halide substitutions. Nitrogenous bases may be conventional bases (A, G, C, T, U), analogs thereof (e.g., inosine or others; see The Biochemistry of the Nucleic Acids 5-36, Adams et al., ed., 11th ed., 1992), derivatives of purines or pyrimidines (e.g., N⁴-methyl deoxyguanosine, deaza- or aza-purines, deaza- or aza-pyrimidines, pyrimidine bases with substituent groups at the 5 or 6 position, purine bases with a substituent at the 2, 6, or 8 positions, 2-amino-6-methylaminopurine, O⁶-methylguanine, 4-thio-pyrimidines, 4-amino-pyrimidines, 4-dimethylhydrazine-pyrimidines, and O⁴-alkyl-pyrimidines; US Pat. No. 5,378,825 and PCT No. WO 93/13121). Nucleic acids may include one or more "abasic" residues where the backbone includes no nitrogenous base for position(s) of the polymer (US Pat. No. 5,585,481). A nucleic acid may comprise only conventional RNA or DNA sugars, bases and linkages, or may include both conventional components and substitutions (e.g., conventional bases with 2' methoxy linkages, or polymers containing both conventional bases and one or more base analogs). Nucleic acid includes "locked nucleic acid" (LNA), an analogue containing one or more LNA nucleotide monomers with a bicyclic furanose unit locked in an RNA mimicking sugar conformation, which enhance hybridization affinity toward complementary RNA and DNA sequences (Vester and Wengel, 2004, Biochemistry 43(42):13233-41). Embodiments of oligomers that may affect stability of a hybridization complex include PNA oligomers, oligomers that include 2'-methoxy or 2'-fluoro substituted RNA, or oligomers that affect the overall charge, charge density, or steric associations of a hybridization complex, including oligomers that contain charged linkages (e.g., phosphorothioates) or neutral groups (e.g., methylphosphonates). 5-methylcytosines may be used in conjunction with any of the foregoing backbones/sugars/linkages including RNA or DNA backbones (or mixtures thereof) unless otherwise indicated. It is understood that when referring to ranges for the length of an oligonucleotide, amplicon, or other nucleic acid, that the range is inclusive of all whole numbers (e.g., 19-25 contiguous nucleotides in length includes 19, 20, 21, 22, 23, 24, and 25).

C residues include methylated and unmethylated cytosines unless the context indicates otherwise. T residues are considered 100% identical to and interchangeable with U residues unless otherwise specified.

An "oligomer" or "oligonucleotide" refers to a nucleic acid of generally less than 1,000 nucleotides (nt), including those in a size range having a lower limit of about 2 to 5 nt and an upper limit of about 500 to 900 nt. Some particular embodiments are oligomers in a size range with a lower limit of about 5 to 15, 16, 17, 18, 19, or 20 nt and an upper limit of about 50 to 600 nt, and other particular embodiments are in a size range with a lower limit of about 10 to 20 nt and an upper limit of about 22 to 100 nt. Oligomers may be purified from naturally occurring sources, but may be synthesized by using any well known enzymatic or chemical method. Oligomers may be referred to by a functional name (e.g., capture probe, primer or promoter primer) but those skilled in the art will understand that such terms refer to oligomers. In some embodiments, an oligomer described herein (e.g., an amplification oligomer described herein) comprises a 3'-OH. In some embodiments, an oligomer described herein (e.g., an amplification or detection oligomer described herein) is made by a process comprising the step of chemically synthesizing the oligomer using sequential solid phase oligonucleotide synthesis.

By "amplicon" or "amplification product" is meant a nucleic acid molecule generated in a nucleic acid amplification reaction and which is derived from a target nucleic acid. An amplicon or amplification product contains a target nucleic acid sequence that may be of the same or opposite sense as the target nucleic acid.

An "amplification oligonucleotide" or "amplification oligomer" refers to an oligonucleotide that hybridizes to a target nucleic acid, or its complement, and participates in a nucleic acid amplification reaction, e.g., serving as a primer or and promoter-primer. Particular amplification oligomers contain at least about 10 contiguous bases, and optionally at least 11, 12, 13, 14, 15, 16, 17, 18, 19, or 20 contiguous bases, that are complementary to a region of the target nucleic acid sequence or its complementary strand. The contiguous bases may be at least about 80%, at least about 90%, or completely complementary to the target sequence to which the amplification oligomer binds. One skilled in the art will understand that the recited ranges include all whole and rational numbers within the range (e.g., 92% or 98.377%). Particular amplification oligomers are about 10 to about 60 bases long and optionally may include modified nucleotides. In some embodiments, an amplification oligomer described herein comprises a 3'-OH.

A "primer" refers to an oligomer that hybridizes to a template nucleic acid and has a 3' end, such as a 3'-OH, that is extended by polymerization. A primer may be optionally modified, e.g., by including a 5' region that is non-complementary to the target sequence. Such modification can include functional additions, such as tags, promoters, or other sequences used or useful for manipulating or amplifying the primer or target oligonucleotide.

"Nucleic acid amplification" refers to any *in vitro* procedure that produces multiple copies of a target nucleic acid sequence, or its complementary sequence, or fragments thereof (i.e., an amplified sequence containing less than the complete target nucleic acid). Examples of nucleic acid amplification procedures include the polymerase chain reaction (PCR) (e.g., US Pat. Nos. 4,683,195, 4,683,202, and 4,800,159), ligase chain reaction (LCR) (e.g., EP Pat. App. 0320308), helicase-dependent amplification (e.g., US Pat. No. 7,282,328), and strand-displacement amplification (SDA) (e.g., US Pat. No. 5,422,252), and transcription associated methods, such as transcription-mediated amplification (TMA), nucleic acid sequence-based amplification (NASBA) and others (e.g., US Pat. Nos. 5,399,491, 5,554,516, 5,437,990, 5,130,238, 4,868,105, and 5,124,246), replicase-mediated amplification (e.g., US Pat. No. 4,786,600). Amplification may be linear or exponential. Replicase-mediated amplification uses self-replicating RNA molecules, and a replicase such as QB-replicase. PCR amplification uses DNA polymerase, primers, and thermal cycling steps to synthesize multiple copies of the two complementary strands of DNA or cDNA. LCR amplification uses at least four separate oligonucleotides to amplify a target and its complementary strand by using multiple cycles of hybridization, ligation, and denaturation. Helicase-dependent amplification uses a helicase to separate the two strands of a DNA duplex generating single-stranded templates, followed by hybridization of sequence-specific primers hybridize to the templates and extension by DNA polymerase to amplify the target sequence. SDA uses a primer that contains a recognition site for a restriction endonuclease that will nick one strand of a hemimodified DNA duplex that includes the target sequence, followed by amplification in a series of primer extension and strand displacement steps. Particular embodiments use PCR or TMA, but it will be apparent to persons of ordinary skill in the art that oligomers disclosed herein may be readily used as primers in other amplification methods.

In cyclic amplification methods that detect amplicons in real-time, the term "Threshold cycle" (Ct) is a measure of the emergence time of a signal associated with amplification of target, and is generally 10x standard deviation of the normalized reporter signal. Once an amplification reaches the "threshold cycle," generally there is considered to be a positive amplification product of a sequence to which the probe binds. The identity of the amplification product can then be determined through methods known to one of skill in the art, such as gel electrophoresis, nucleic acid sequencing, and other such analytical procedures.

As used herein, the term "relative fluorescence unit" ("RFU") is a unit of measurement of fluorescence intensity. RFU varies with the characteristics of the detection means used for the measurement, and can be used as a measurement to compare relative intensities between samples and controls. The analytical sensitivity (limit of detection or LoD) is typically determined in terms of CFU (colony forming units) per mL or other unit volume or per reaction.

"Detection probe" or "probe" refers to an oligomer that hybridizes specifically to a target sequence, including an amplified sequence, under conditions that promote nucleic acid hybridization, for detection of the target nucleic acid. Detection may either be direct (i.e., probe hybridized directly to the target) or indirect (i.e., a probe hybridized to an intermediate structure that links the probe to the target). A probe's target sequence generally refers to the specific sequence within a larger sequence which the probe hybridizes specifically. A detection probe may include target-specific sequences and a non-target-complementary sequence. Such non-target-complementary sequences can include sequences which will confer a desired secondary or tertiary structure, such as a hairpin structure, which can be used to facilitate detection and/or amplification (e.g., US Pat. Nos. 5,118,801, 5,312,728, 6,835,542, and 6,849,412). Probes of a defined sequence may be produced by techniques known to those of ordinary skill in the art, such as by chemical synthesis, and by in vitro or in vivo expression from recombinant nucleic acid molecules.

By "hybridization" or "hybridize" is meant the ability of two completely or partially complementary nucleic acid strands to come together under specified hybridization assay conditions in a parallel or antiparallel orientation to form a stable structure having a double-stranded region. The two constituent strands of this double-stranded structure, sometimes called a hybrid, are held together by hydrogen bonds. Although these hydrogen bonds most commonly form between nucleotides containing the bases adenine and thymine or uracil (A and T or U) or cytosine and guanine (C and G) on single nucleic acid strands, base pairing can also form between bases which are not members of these "canonical" pairs. Non-canonical base pairing is well-known in the art. (See, e.g., R. L. P. Adams et al., The Biochemistry of the Nucleic Acids (11th ed. 1992).)

By "preferentially hybridize" is meant that under stringent hybridization conditions, an amplification or detection probe oligomer can hybridize to its target nucleic acid to form stable oligomer:target hybrid, but not form a sufficient number of stable oligomer:non-target hybrids. Amplification and detection oligomers that preferentially hybridize to a target nucleic acid are useful to amplify and detect target nucleic acids, but not non-targeted organisms, especially phylogenetically closely related organisms. Thus, the oligomer hybridizes to target nucleic acid to a sufficiently greater extent than to non-target nucleic acid to enable one having ordinary skill in the art to accurately amplify and/or detect the presence (or absence) of nucleic acid derived from the specified influenza viruses as appropriate. In general, reducing the degree of complementarity between an oligonucleotide sequence and its target sequence will decrease the degree or rate of hybridization of the oligonucleotide to its target region. However, the inclusion of one or more non-complementary nucleosides or nucleobases may facilitate the ability of an oligonucleotide to discriminate against non-target organisms.

Preferential hybridization can be measured using techniques known in the art and described herein, such as in the examples provided below. In some embodiments, there is at least a 10-fold difference between target and non-target hybridization signals in a test sample, at least a 100-fold difference, or at least a 1,000-fold difference. In some embodiments, non-target hybridization signals in a test sample are no more than the background signal level.

By "stringent hybridization conditions," or "stringent conditions" is meant conditions permitting an oligomer to preferentially hybridize to a target nucleic acid (such as a GAS nucleic acid) and not to nucleic acid derived from a closely related non-target nucleic acid. While the definition of stringent hybridization conditions does not vary, the actual reaction environment that can be used for stringent hybridization may vary depending upon factors including the GC content and length of the oligomer, the degree of similarity between the oligomer sequence and sequences of non-target nucleic acids that may be present in the test sample, and the target sequence. Hybridization conditions include the temperature and the composition of the hybridization reagents or solutions. Exemplary hybridization assay conditions for amplifying and/or detecting target nucleic acids derived from one or more strains of GAS with the oligomers of the present disclosure correspond to a temperature of about 60 °C when the salt concentration, such as a monovalent salt, e.g., KCl, is in the range of about 0.6-0.9 M. Specific hybridization assay conditions are set forth infra in the Examples section. Other acceptable stringent hybridization conditions could be easily ascertained by those having ordinary skill in the art.

By "competes for hybridization to a GAS nucleic acid under stringent conditions" with a referenced oligomer is meant that an oligomer substantially reduces the binding of the referenced oligomer to its target GAS sequence under stringent conditions, the competing oligomer when supplied in excess can reduce binding of the referenced oligomer at a sub-saturating concentration by about 20%, 30%, 40%, 50%, or more, or the Tm of the competing oligomer is higher than or within about 5, 4, 3, 2, or 1°C of the Tm of the referenced oligomer to the target. Suitable oligonucleotide competition assay conditions and procedures are known in the art.

By "assay conditions" is meant conditions permitting stable hybridization of an oligonucleotide to a target nucleic acid. Assay conditions do not require preferential hybridization of the oligonucleotide to the target nucleic acid.

"Label" or "detectable label" refers to a moiety or compound joined directly or indirectly to a probe that is detected or leads to a detectable signal. Direct joining may use covalent bonds or non-covalent interactions (e.g., hydrogen bonding, hydrophobic or ionic interactions, and chelate or coordination complex formation) whereas indirect joining may use a bridging moiety or linker (e.g., via an antibody or additional oligonucleotide(s), which amplify a detectable signal. Any detectable moiety may be used, e.g., radionuclide, ligand such as biotin or avidin, enzyme, enzyme substrate, reactive group, chromophore such as a dye or particle (e.g., latex or metal bead) that imparts a detectable color, luminescent compound (e.g. bioluminescent, phosphorescent, or chemiluminescent compound), and fluorescent compound (i.e., fluorophore). Embodiments of fluorophores include those that absorb light in the range of about 495 to 650 nm and emit light in the range of about 520 to 670 nm, which include those known as FAM^{™}, TET^{™}, CAL FLUOR^{™} (Orange or Red), and QUASAR^{™} compounds. Fluorophores may be used in combination with a quencher molecule that absorbs light when in close proximity to the fluorophore to diminish background fluorescence. Such quenchers are well known in the art and include, e.g., BLACK HOLE QUENCHER^{™} (or BHQ^{™}), TAMRA^{™} compounds, or BLACK BERRY QUENCHERS^{™} (or BBQ^{™}). Particular embodiments include a "homogeneous detectable label" that is detectable in a homogeneous system in which bound labeled probe in a mixture exhibits a detectable change compared to unbound labeled probe, which allows the label to be detected without physically removing hybridized from unhybridized labeled probe (e.g., US Pat. Nos. 5,283,174, 5,656,207, and 5,658,737). Particular homogeneous detectable labels include chemiluminescent compounds, including acridinium ester ("AE") compounds, such as standard AE or AE derivatives which are well known (US Pat. Nos. 5,656,207, 5,658,737, and 5,639,604). Methods of synthesizing labels, attaching labels to nucleic acid, and detecting signals from labels are well known (e.g., Sambrook et al., Molecular Cloning, A Laboratory Manual, 2nd ed. (Cold Spring Harbor Laboratory Press, Cold Spring Harbor, NY, 1989) at Chapt. 10, and US Pat. Nos. 5,658,737, 5,656,207, 5,547,842, 5,283,174, and 4,581,333, and EP Pat. App. 0 747 706). Particular methods of linking an AE compound to a nucleic acid are known (e.g., US Pat. No. 5,585,481 and US Pat. No. 5,639,604, see column 10, line 6 to column 11, line 3, and Example 8). Particular AE labeling positions are a probe's central region and near a region of A/T base pairs, at a probe's 3' or 5' terminus, or at or near a mismatch site with a known sequence that is the probe should not detect compared to the desired target sequence. Other detectably labeled probes include TaqMan^{™} probes, molecular torches, and molecular beacons. TaqMan^{™} probes include a donor and acceptor label wherein fluorescence is detected upon enzymatically degrading the probe during amplification in order to release the fluorophore from the presence of the quencher. Molecular torches and beacons exist in open and closed configurations wherein the closed configuration quenches the fluorophore and the open position separates the fluorophore from the quencher to allow fluorescence. Hybridization to target opens the otherwise closed probes.

Sequences are "sufficiently complementary" if they allow stable hybridization of two nucleic acid sequences, e.g., stable hybrids of probe and target sequences, although the sequences need not be completely complementary. That is, a "sufficiently complementary" sequence that hybridizes to another sequence by hydrogen bonding between a subset series of complementary nucleotides by using standard base pairing (e.g., G:C, A:T, or A:U), although the two sequences may contain one or more residues (including abasic positions) that are not complementary so long as the entire sequences in appropriate hybridization conditions to form a stable hybridization complex. Sufficiently complementary sequences may be at least about 80%, at least about 90%, or completely complementary in the sequences that hybridize together. Appropriate hybridization conditions are well known to those skilled in the art, can be predicted based on sequence composition, or can be determined empirically by using routine testing (e.g., Sambrook et al., Molecular Cloning, A Laboratory Manual, 2nd ed. at §§ 1.90-1.91, 7.37-7.57, 9.47-9.51 and 11.47-11.57, particularly §§ 9.50-9.51, 11.12-11.13, 11.45-11.47 and 11.55-11.57).

A "non-extendable" oligomer includes a blocking moiety at or near its 3'-terminus to prevent extension. A blocking group near the 3' end is in some embodiments within five residues of the 3' end and is sufficiently large to limit binding of a polymerase to the oligomer, and other embodiments contain a blocking group covalently attached to the 3' terminus. Many different chemical groups may be used to block the 3' end, e.g., alkyl groups, non-nucleotide linkers, alkane-diol dideoxynucleotide residues, and cordycepin. Further examples of blocking moieties include a 3'-deoxy nucleotide (e.g., a 2',3'-dideoxy nucleotide); a 3'-phosphorylated nucleotide; a fluorophore, quencher, or other label that interferes with extension; an inverted nucleotide (e.g., linked to the preceding nucleotide through a 3'-to-3' phosphodiester, optionally with an exposed 5'-OH or phosphate); or a protein or peptide joined to the oligonucleotide so as to prevent further extension of a nascent nucleic acid chain by a polymerase. A non-extendable oligonucleotide of the present disclosure may be at least 10 bases in length, and may be up to 15, 20, 25, 30, 35, 40, 50 or more nucleotides in length. Non-extendable oligonucleotides that comprise a detectable label can be used as probes.

References, particularly in the claims, to "the sequence of SEQ ID NO: X" refer to the base sequence of the corresponding sequence listing entry and do not require identity of the backbone (e.g., RNA, 2'-O-Me RNA, or DNA) or base modifications (e.g., methylation of cytosine residues) unless otherwise indicated.

Unless defined otherwise, all scientific and technical terms used herein have the same meaning as commonly understood by those skilled in the relevant art. General definitions may be found in technical books relevant to the art of molecular biology, e.g., DICTIONARY OF MICROBIOLOGY AND MOLECULAR BIOLOGY, 2nd ed. (Singleton et al., 1994, John Wiley & Sons, New York, NY) or THE HARPER COLLINS DICTIONARY OF BIOLOGY (Hale & Marham, 1991, Harper Perennial, New York, NY).

### Exemplary compositions, kits, methods, and uses

The present disclosure provides oligomers, compositions, and kits, useful for amplifying or detecting GAS in a sample. In some embodiments, compositions and methods disclosed herein are capable of detecting GAS with a high degree of sensitivity and specificity compared to existing compositions and methods.

In some embodiments, oligomers are provided, e.g., in a kit or composition. Oligomers generally comprise a target-hybridizing region, e.g., configured to hybridize specifically to a GAS nucleic acid. While oligomers of different lengths and base composition may be used for amplifying GAS nucleic acids, in some embodiments oligomers in this disclosure have target-hybridizing regions from about 10-60 bases in length, about 14-50 bases in length, about 14-40 bases in length, about 14-35 bases in length, or about 15-30 bases in length. In some embodiments, an oligomer comprises a second region of sequence in addition to the target-hybridizing region, such as a promoter, which can be located 5' of the target-hybridizing region. In some embodiments, an oligomer does not comprise a second region of sequence.

In some embodiments, a pair of oligomers is provided wherein one oligomer is configured to hybridize to a sense strand of a GAS nucleic acid and the other is configured to hybridize to an anti-sense strand of a GAS nucleic acid. Such oligomers include primer pairs for PCR or other forms of amplification.

In some embodiments, one or more oligomers, such as a primer pair or a primer pair and a third oligomer which is optionally labeled (e.g., for use as a probe), are configured to hybridize to a GAS target sequence. In some embodiments, a plurality of oligomers, such as a plurality of primers (including one or more pairs) or a primer pair, optional additional primers, and a plurality of third oligomers which are optionally labeled (e.g., for use as a probe), are provided which collectively hybridize to target sequences of a plurality of GAS strains. In some embodiments, the plurality of GAS strains includes strain NCTC 8709. Strain NCTC 8709 is available form Public Health England, Salisbury, UK.

In some embodiments, one or more oligomers comprise an inosine.

In some embodiments, a composition or kit comprises a forward amplification oligomer comprising the sequence of any one of SEQ ID NOs: 1, 4, 7, 10, 13, 16, 16, 16, 19, 22, 27, 30, 31, 34, 37. In some embodiments, a composition or kit comprises a forward amplification oligomer comprising the sequence of any one of the foregoing SEQ ID NOs with up to one mismatch. In some embodiments, a composition or kit comprises a forward amplification oligomer comprising the sequence of any one of the foregoing SEQ ID NOs with up to two mismatches. In some embodiments, a composition or kit comprises a forward amplification oligomer that competes for hybridization to a GAS nucleic acid under stringent conditions with an amplification oligomer whose sequence consists of the sequence of any one of the foregoing SEQ ID NOs. In some embodiments, a composition or kit comprises a forward amplification oligomer that comprises at least 10, 11, 12, 13, 14, 15, 16, 17, 18, 19, or 20 contiguous nucleotides of the sequence of any one of the foregoing SEQ ID NOs. In any of the foregoing embodiments, the composition or kit may further comprise a reverse amplification oligomer which, together with the forward amplification oligomer, is configured to generate an amplicon under amplification conditions in the presence of a GAS target nucleic acid. In some embodiments, the reverse amplification oligomer is an amplification oligomer disclosed herein, which is configured together with the forward amplification oligomer to generate an amplicon under amplification conditions in the presence of a GAS target nucleic acid. In some embodiments, the kit or composition further comprises a probe oligomer configured to hybridize to an amplicon generated using the forward amplification oligomer, e.g., a probe oligomer disclosed herein.

In some embodiments, a composition or kit comprises a reverse amplification oligomer comprising the sequence of any one of SEQ ID NOs: 3, 6, 9, 12, 15, 18, 17, 20, 23, 28, 28, 32, 35, or 38. In some embodiments, a composition or kit comprises a reverse amplification oligomer comprising the sequence of any one of the foregoing SEQ ID NOs with up to one mismatch. In some embodiments, a composition or kit comprises a reverse amplification oligomer comprising the sequence of any one of the foregoing SEQ ID NOs with up to two mismatches. In some embodiments, a composition or kit comprises a reverse amplification oligomer that competes for hybridization to a GAS nucleic acid under stringent conditions with an amplification oligomer whose sequence consists of the sequence of any one of the foregoing SEQ ID NOs. In some embodiments, a composition or kit comprises a reverse amplification oligomer that comprises at least 10, 11, 12, 13, 14, 15, 16, 17, 18, 19, or 20 contiguous nucleotides of the sequence of any one of the foregoing SEQ ID NOs. In any of the foregoing embodiments, the composition or kit may further comprise a forward amplification oligomer which, together with the reverse amplification oligomer, is configured to generate an amplicon under amplification conditions in the presence of a GAS target nucleic acid. In some embodiments, the forward amplification oligomer is an amplification oligomer disclosed herein, which is configured together with the forward amplification oligomer to generate an amplicon under amplification conditions in the presence of a GAS target nucleic acid. In some embodiments, the kit or composition further comprises a probe oligomer configured to hybridize to an amplicon generated using the reverse amplification oligomer, e.g., a probe oligomer disclosed herein.

In some embodiments, a composition or kit comprises an oligomer (e.g., suitable for use as a probe, and which may be optionally labeled) comprising the sequence of any one of SEQ ID NOs: 2, 5, 8, 11, 14, 18, 25, 26, 21, 24, 29, 29, 33, 36, 39. In some embodiments, a composition or kit comprises an oligomer (e.g., suitable for use as a probe, and which may be optionally labeled) comprising the sequence of any one of the foregoing SEQ ID NOs with up to one mismatch. In some embodiments, a composition or kit comprises an oligomer (e.g., suitable for use as a probe, and which may be optionally labeled) comprising the sequence of any one of the foregoing SEQ ID NOs with up to two mismatches. In some embodiments, a composition or kit comprises an oligomer (e.g., suitable for use as a probe, and which may be optionally labeled) that competes for hybridization to a GAS nucleic acid under stringent conditions with an amplification oligomer whose sequence consists of the sequence of any one of the foregoing SEQ ID NOs. In some embodiments, a composition or kit comprises an oligomer (e.g., suitable for use as a probe, and which may be optionally labeled) that comprises at least 10, 11, 12, 13, 14, 15, 16, 17, 18, 19, or 20 contiguous nucleotides of the sequence of any one of the foregoing SEQ ID NOs. In any of the foregoing embodiments, the composition or kit may further comprise a forward and/or reverse amplification oligomer configured to generate an amplicon under amplification conditions in the presence of a GAS target nucleic acid to which the oligomer (e.g., suitable for use as a probe, and which may be optionally labeled) is configured to hybridize. In some embodiments, the forward and/or reverse amplification oligomers are amplification oligomers disclosed herein.

Exemplary primer pairs and optional third oligomers suitable for use therewith are set forth in the following table.

**Table 1. Exemplary oligomer sets. Oligomers are referred to by their SEQ ID NO (see the Sequence Table below).**

| Oligomer 1 (e.g., forward primer) | Optional Oligomer 3 (optionally labeled, e.g., probe) | Oligomer 2 (e.g., reverse primer) |
|---|---|---|
| 1 | 2 | 3 |
| 4 | 5 | 6 |
| 7 | 8 | 9 |
| 10 | 11 | 12 |
| 13 | 14 | 12, 15, or 12 and 15 |
| 16 | 25 | 17 |
| 16 | 26 | 17 |
| 16 | 18 | 17 |
| 19 | 21 | 20 |
| 22 | 24 | 23 |
| 27 | 29 | 28 |
| 30 | 29 | 28 |
| 31 | 33 | 32 |
| 34 | 36 | 35 |
| 37 | 39 | 38 |

In some embodiments, a kit or composition comprises a pair of an oligomer 1 and oligomer 2 shown in Table 1, or a set of oligomers 1-3 shown in Table 1. In some embodiments, a kit or composition comprises a pair of an oligomer 1 and oligomer 2, or a set of oligomers 1-3, comprising the sequences indicated for a set in Table 1 with up to one mismatch, or wherein one or two of the oligomers in the set comprise the sequence indicated for a set in Table 1 with up to one mismatch and the remaining oligomer(s) comprise the sequence(s) indicated for the same set in Table 1. In some embodiments, a kit or composition comprises a pair of an oligomer 1 and oligomer 2, or a set of oligomers 1-3, comprising the sequences indicated for a set in Table 1 with up to two mismatches, or wherein one or two of the oligomers in the set comprise the sequence indicated for a set in Table 1 with up to two mismatches and the remaining oligomer(s) comprise the sequence(s) indicated for the same set in Table 1. In some embodiments, a kit or composition comprises a pair of an oligomer 1 and oligomer 2, or a set of oligomers 1-3, that respectively compete for hybridization to a GAS nucleic acid under stringent conditions with oligomers whose sequences consist of the sequences of oligomers 1-2 or 1-3 of a set in Table 1. In some embodiments, a kit or composition comprises a pair of an oligomer 1 and oligomer 2, or a set of oligomers 1-3, wherein one or two of the oligomers in the set compete for hybridization to a GAS nucleic acid under stringent conditions with oligomers whose sequences consist of the sequences of oligomers of a set in Table 1 and the remaining oligomer(s) comprise the sequence(s) indicated for the same set in Table 1. In some embodiments, a kit or composition comprises a pair of an oligomer 1 and oligomer 2, or a set of oligomers 1-3, that respectively comprise at least 10, 11, 12, 13, 14, 15, 16, 17, 18, 19, or 20 contiguous nucleotides of the sequences of oligomers 1-2 or 1-3 of a set in Table 1. In some embodiments, a kit or composition comprises a pair of an oligomer 1 and oligomer 2, or a set of oligomers 1-3, wherein one or two of the oligomers in the set comprise at least 10, 11, 12, 13, 14, 15, 16, 17, 18, 19, or 20 contiguous nucleotides of the sequences of oligomers of a set in Table 1 and the remaining oligomer(s) comprise the sequence(s) indicated for the same set in Table 1.

In some embodiments, one or more oligomers comprises a 5-methyl-deoxycytosine (5-me-dC) residue. For example, oligomers corresponding to one or more of SEQ ID NOs: 4, 5, 6, 16, 17, 18, 23, 25, and 26, if present, may comprise one or more 5-me-dC residues. As used herein, an oligomer "corresponds" to a given SEQ ID NO if any of the following are true: it comprises the sequence of the SEQ ID NO; it comprises a sequence with 0, 1, or 2 mismatches to the SEQ ID NO; it competes for hybridization to a Group A Streptococcus (GAS) nucleic acid under stringent conditions with an amplification oligomer whose sequence consists of the SEQ ID NO; or it comprises at least 10, 11, 12, 13, 14, 15, 16, 17, 18, 19, or 20 contiguous nucleotides of a first sequence which is the SEQ ID NO. In some embodiments, an oligomer corresponding to SEQ ID NO: 4 comprises one or more 5-methyl-dC residues comprise residues corresponding to one or more, or all, of positions 8, 18, and 24 of SEQ ID NO: 4. As used herein, a residue corresponds to a given position of a SEQ ID NO if it aligns to that position when compared with a standard sequence alignment algorithm, e.g., the Smith-Waterman algorithm. In some embodiments, an oligomer corresponding to SEQ ID NO: 5 comprises one or more 5-methyl-dC residues comprise residues corresponding to one or more, or all, of positions 2, 4, 15, 19, and 21 of SEQ ID NO: 5. In some embodiments, an oligomer corresponding to SEQ ID NO: 6 comprises one or more 5-methyl-dC residues comprise residues corresponding to one or more, or all, of positions 7, 8, 11, and 13 of SEQ ID NO: 6. In some embodiments, an oligomer corresponding to SEQ ID NO: 16 comprises one or more 5-methyl-dC residues comprise residues corresponding to one or more, or all, of positions 7, 8, 10, and 21 of SEQ ID NO: 16. In some embodiments, an oligomer corresponding to SEQ ID NO: 17 comprises one or more 5-methyl-dC residues comprise residues corresponding to one or more, or all, of positions 6, 7, 9, 11, 12, 15, and 19 of SEQ ID NO: 17. In some embodiments, an oligomer corresponding to SEQ ID NO: 18 comprises one or more 5-methyl-dC residues comprise residues corresponding to one or more, or all, of positions 17 and 23 of SEQ ID NO: 18. In some embodiments, an oligomer corresponding to SEQ ID NO: 23 comprises one or more 5-methyl-dC residues comprise residues corresponding to one or more, or all, of positions 5, 7, 11, 13, and 19 of SEQ ID NO: 23. In some embodiments, an oligomer corresponding to SEQ ID NO: 25 comprises one or more 5-methyl-dC residues comprise residues corresponding to one or more, or all, of positions 17, 23, 25, 26, and 28 of SEQ ID NO: 25. In some embodiments, an oligomer corresponding to SEQ ID NO: 26 comprises one or more 5-methyl-dC residues comprise residues corresponding to one or more, or all, of positions 8, 14, 15, 16, 17, and 28 of SEQ ID NO: 26. In any of the foregoing embodiments, the oligomer corresponding to the indicated SEQ ID NO optionally comprises the sequence of the SEQ ID NO.

Where a labeled oligomer is provided, in some embodiments, the label is a non-nucleotide label. Suitable labels include compounds that emit a detectable light signal, e.g., fluorophores or luminescent (e.g., chemiluminescent) compounds that can be detected in a homogeneous mixture. More than one label, and more than one type of label, may be present on a particular probe, or detection may rely on using a mixture of probes in which each probe is labeled with a compound that produces a detectable signal (see. e.g., U.S. Pat. Nos. 6,180,340 and 6,350,579, each incorporated by reference herein). Labels may be attached to a probe by various means including covalent linkages, chelation, and ionic interactions, but in some embodiments the label is covalently attached. For example, in some embodiments, a detection probe has an attached chemiluminescent label such as, e.g., an acridinium ester (AE) compound (see. e.g., U.S. Pat. Nos. 5,185,439; 5,639,604; 5,585,481; and 5,656,744). A label, such as a fluorescent or chemiluminescent label, can be attached to the probe by a non-nucleotide linker (see. e.g., U.S. Pat. Nos. 5,585,481; 5,656,744; and 5,639,604).

In some embodiments, a probe (e.g., comprising a fluorescent label) further comprises a second label that interacts with the first label. For example, the second label can be a quencher. Such probes can be used, e.g., in TaqMan^{™} assays, where hybridization of the probe to a target or amplicon followed by nucleolysis by a polymerase comprising 5'-3' exonuclease activity results in liberation of the fluorescent label and thereby increased fluorescence, or fluorescence independent of the interaction with the second label.

Examples of interacting donor/acceptor label pairs that may be used in connection with the disclosure, making no attempt to distinguish FRET from non-FRET pairs, include fluorescein/tetramethylrhodamine, IAEDANS/ fluororescein, EDANS/DABCYL, coumarin/DABCYL, fluorescein/fluorescein, BODIPY FL/BODIPY FL, fluorescein/DABCYL, lucifer yellow/DABCYL, BODIPY/DABCYL, eosine/DABCYL, erythrosine/DABCYL, tetramethylrhodamine/DABCYL, Texas Red/DABCYL, CY5/BH1, CY5/BH2, CY3/BH1, CY3/BH2 and fluorescein/QSY7 dye. Those having an ordinary level of skill in the art will understand that when donor and acceptor dyes are different, energy transfer can be detected by the appearance of sensitized fluorescence of the acceptor or by quenching of donor fluorescence. Non-fluorescent acceptors such as DABCYL and the QSY7 dyes advantageously eliminate the potential problem of background fluorescence resulting from direct (i.e., non-sensitized) acceptor excitation. Exemplary fluorophore moieties that can be used as one member of a donor-acceptor pair include fluorescein, ROX, and the CY dyes (such as CY5). Exemplary quencher moieties that can be used as another member of a donor-acceptor pair include DABCYL and the BLACK HOLE QUENCHER moieties which are available from Biosearch Technologies, Inc., (Novato, Calif.).

In some embodiments, a labeled oligomer (e.g., probe) is non-extendable. For example, the labeled oligomer can be rendered non-extendable by 3'-phosphorylation, having a 3'-terminal 3'-deoxynucleotide (e.g., a terminal 2',3'-dideoxynucleotide), having a 3'-terminal inverted nucleotide (e.g., in which the last nucleotide is inverted such that it is joined to the penultimate nucleotide by a 3' to 3' phosphodiester linkage or analog thereof, such as a phosphorothioate), or having an attached fluorophore, quencher, or other label that interferes with extension (possibly but not necessarily attached via the 3' position of the terminal nucleotide). In some embodiments, the 3'-terminal nucleotide is not methylated.

Also provided by the disclosure is a reaction mixture for determining the presence or absence of a GAS target nucleic acid or quantifying the amount thereof in a sample. A reaction mixture in accordance with the present disclosure comprises at least one or more of the following: an oligomer combination as described herein for amplification of a GAS target nucleic acid; and a detection probe oligomer as described herein for determining the presence or absence of a GAS amplification product. The reaction mixture may further include a number of optional components such as, for example, capture probes, e.g., poly-(k) capture probes as described in US 2013/0209992, which is incorporated herein by reference. For an amplification reaction mixture, the reaction mixture will typically include other reagents suitable for performing in vitro amplification such as, e.g., buffers, salt solutions, appropriate nucleotide triphosphates (e.g., dATP, dCTP, dGTP, and dTTP; and/or ATP, CTP, GTP and UTP), and/or enzymes (e.g., a thermostable DNA polymerase, or reverse transcriptase and/or RNA polymerase), and will typically include test sample components, in which a GAS target nucleic acid may or may not be present. A reaction mixture may include amplification oligomers for only one target region of a GAS genome, or it may include amplification oligomers for multiple GAS target regions of the same or different strains, e.g., NCTC 8709, NCTC 12696 (available from Public Health England, Salisbury, UK), CDC-DD1147 (available from American Type Culture Collection, Manassas, VA), and/or ATCC BAA-946 (available from American Type Culture Collection, Manassas, VA). In addition, for a reaction mixture that includes a detection probe together with an amplification oligomer combination, selection of amplification oligomers and detection probe oligomers for a reaction mixture are linked by a common target region (i.e., the reaction mixture will include a probe that binds to a sequence amplifiable by an amplification oligomer combination of the reaction mixture).

In some embodiments, the reaction mixture comprises KCl. In some embodiments, the KCl concentration is about 50 mM. In some embodiments, the KCl concentration is greater than about 50 mM, e.g., about 60-150 mM, about 75-125 mM, about 80-120 mM, about 85-115 mM, or about 90-110 mM. In some embodiments, the KCl concentration is 55-65, 65-75, 75-85, 85-95, 95-105, 105-115, 115-125, 125-135, or 135-145, wherein each of the foregoing is in mM and is optionally modified by "about". In some embodiments, a composition according to the disclosure comprises KCl, e.g., at any of the foregoing concentrations. In some embodiments, a method according to the disclosure comprises performing an amplification reaction in the presence of KCl, e.g., at any of the foregoing concentrations.

Also provided by the subject disclosure are kits for practicing the methods as described herein. A kit in accordance with the present disclosure comprises at least one or more of the following: an amplification oligomer combination as described herein for amplification of a GAS target nucleic acid; and at least one detection probe oligomer as described herein for determining the presence or absence of a GAS amplification product. In some embodiments, any oligomer combination described herein is present in the kit. The kits may further include a number of optional components such as, for example, capture probes such as those described herein. Other reagents that may be present in the kits include reagents suitable for performing in vitro amplification such as, e.g., buffers, salt solutions, appropriate nucleotide triphosphates (e.g., dATP, dCTP, dGTP, dTTP; and/or ATP, CTP, GTP and UTP), and/or enzymes (e.g., a thermostable DNA polymerase, or a reverse transcriptase and/or RNA polymerase). Oligomers as described herein may be packaged in a variety of different embodiments, and those skilled in the art will appreciate that the disclosure embraces many different kit configurations. For example, a kit may include amplification oligomers for only one target region of a GAS genome, or it may include amplification oligomers for multiple GAS target regions of the same or different strains as discussed above. In addition, for a kit that includes a detection probe together with an amplification oligomer combination, selection of amplification oligomers and detection probe oligomers for a kit are linked by a common target region (i.e., the kit will include a probe that binds to a sequence amplifiable by an amplification oligomer combination of the kit). In certain embodiments, the kit further includes a set of instructions for practicing methods in accordance with the present disclosure, where the instructions may be associated with a package insert and/or the packaging of the kit or the components thereof.

Any method disclosed herein is also to be understood as a disclosure of corresponding uses of materials involved in the method directed to the purpose of the method. Any of the oligomers comprising GAS sequence and any combinations (e.g., kits and compositions) comprising such an oligomer are to be understood as also disclosed for use in detecting GAS, and for use in the preparation of a composition for detecting GAS.

Broadly speaking, methods can comprise one or more of the following components: target capture, in which GAS nucleic acid (e.g., from a sample, such as a clinical sample) is annealed to a capture oligomer; isolation, e.g., washing, to remove material not associated with a capture oligomer; amplification; and amplicon detection, which may be performed in real time with amplification. Certain embodiments involve each of the foregoing steps. Certain embodiments involve exponential amplification, optionally with a preceding linear amplification step. Certain embodiments involve exponential amplification and amplicon detection. Certain embodiments involve any two of the components listed above, optionally wherein one of the components is amplification. Certain embodiments involve any two components listed adjacently above, e.g., washing and amplification, or amplification and detection.

In some embodiments, amplification comprises contacting the sample with at least two oligomers for amplifying a GAS nucleic acid target region corresponding to a GAS target nucleic acid, where the oligomers include at least two amplification oligomers as described above (e.g., one or more oriented in the sense direction and one or more oriented in the antisense direction for exponential amplification); (2) performing an in vitro nucleic acid amplification reaction, where any GAS target nucleic acid present in the sample is used as a template for generating an amplification product; and (3) detecting the presence or absence of the amplification product, thereby determining the presence or absence of GAS in the sample, or quantifying the amount of GAS nucleic acid in the sample.

A detection method in accordance with the present disclosure can further include the step of obtaining the sample to be subjected to subsequent steps of the method. In certain embodiments, "obtaining" a sample to be used includes, for example, receiving the sample at a testing facility or other location where one or more steps of the method are performed, and/or retrieving the sample from a location (e.g., from storage or other depository) within a facility where one or more steps of the method are performed.

In certain embodiments, the method further includes purifying the GAS target nucleic acid from other components in the sample, e.g., before an amplification, such as before a capture step. Such purification may include methods of separating and/or concentrating organisms contained in a sample from other sample components, or removing or degrading non-nucleic acid sample components, e.g., protein, carbohydrate, salt, lipid, etc. In some embodiments, DNA in the sample is degraded, e.g., with DNase, and optionally removing or inactivating the DNase or removing degraded DNA.

In particular embodiments, purifying the target nucleic acid includes capturing the target nucleic acid to specifically or non-specifically separate the target nucleic acid from other sample components. Non-specific target capture methods may involve selective precipitation of nucleic acids from a substantially aqueous mixture, adherence of nucleic acids to a support that is washed to remove other sample components, or other means of physically separating nucleic acids from a mixture that contains GAS nucleic acid and other sample components.

In some embodiments, target capture occurs in a solution phase mixture that contains one or more capture probe oligomers that hybridize to the GAS target sequence under hybridizing conditions. For embodiments comprising a capture probe tail, the GAS-target:capture-probe complex is captured by adjusting the hybridization conditions so that the capture probe tail hybridizes to the immobilized probe. Certain embodiments use a particulate solid support, such as paramagnetic beads.

Isolation can follow capture, wherein the complex on the solid support is separated from other sample components. Isolation can be accomplished by any appropriate technique, e.g., washing a support associated with the GAS-target-sequence one or more times (e.g., 2 or 3 times) to remove other sample components and/or unbound oligomer. In embodiments using a particulate solid support, such as paramagnetic beads, particles associated with the GAS-target may be suspended in a washing solution and retrieved from the washing solution, In some embodiments by using magnetic attraction. To limit the number of handling steps, the GAS target nucleic acid may be amplified by simply mixing the GAS target sequence in the complex on the support with amplification oligomers and proceeding with amplification steps.

Exponentially amplifying a GAS target sequence utilizes an in vitro amplification reaction using at least two amplification oligomers that flank a target region to be amplified. In some embodiments, at least first and second oligomers as described above are provided. In some embodiments, a plurality of pairs of oligomers is provided, wherein the plurality comprises oligomer pairs configured to hybridize to at least two GAS strains, e.g., MGAS 10394 and NCTC 8709. The amplification reaction can be cycled or isothermal. Suitable amplification methods include, for example, replicase-mediated amplification, polymerase chain reaction (PCR), ligase chain reaction (LCR), strand-displacement amplification (SDA), and transcription-mediated or transcription-associated amplification (TMA).

A detection step may be performed using any of a variety of known techniques to detect a signal specifically associated with the amplified target sequence, such as, e.g., by hybridizing the amplification product with a labeled detection probe and detecting a signal resulting from the labeled probe (including from label released from the probe following hybridization in some embodiments). In some embodiments, the labeled probe comprises a second moiety, such as a quencher or other moiety that interacts with the first label, as discussed above. The detection step may also provide additional information on the amplified sequence, such as, e.g., all or a portion of its nucleic acid base sequence. Detection may be performed after the amplification reaction is completed, or may be performed simultaneously with amplifying the target region, e.g., in real time. In one embodiment, the detection step allows homogeneous detection, e.g., detection of the hybridized probe without removal of unhybridized probe from the mixture (see. e.g., U.S. Pat. Nos. 5,639,604 and 5,283,174). In some embodiments, the nucleic acids are associated with a surface that results in a physical change, such as a detectable electrical change. Amplified nucleic acids may be detected by concentrating them in or on a matrix and detecting the nucleic acids or dyes associated with them (e.g., an intercalating agent such as ethidium bromide or cyber green), or detecting an increase in dye associated with nucleic acid in solution phase. Other methods of detection may use nucleic acid detection probes that are configured to specifically hybridize to a sequence in the amplified product and detecting the presence of the probe:product complex, or by using a complex of probes that may amplify the detectable signal associated with the amplified products (e.g., U.S. Pat. Nos. 5,424,413; 5,451,503; and 5,849,481; each incorporated by reference herein). Directly or indirectly labeled probes that specifically associate with the amplified product provide a detectable signal that indicates the presence of the target nucleic acid in the sample. In particular, the amplified product will contain a target sequence in or complementary to a sequence in the GAS genomic DNA, and a probe will bind directly or indirectly to a sequence contained in the amplified product to indicate the presence of GAS nucleic acid in the tested sample.

In embodiments that detect the amplified product near or at the end of the amplification step, a linear detection probe may be used to provide a signal to indicate hybridization of the probe to the amplified product. One example of such detection uses a luminescentally labeled probe that hybridizes to target nucleic acid. Luminescent label is then hydrolyzed from non-hybridized probe. Detection is performed by chemiluminescence using a luminometer (see, e.g., International Patent Application Pub. No. WO 89/002476). In other embodiments that use real-time detection, the detection probe may be a hairpin probe such as, for example, a molecular beacon, molecular torch, or hybridization switch probe that is labeled with a reporter moiety that is detected when the probe binds to amplified product. Such probes may comprise target-hybridizing sequences and non-target-hybridizing sequences. Various forms of such probes are described, e.g., in U.S. Pat. Nos. 5,118,801; 5,312,728; 5,925,517; 6,150,097; 6,849,412; 6,835,542; 6,534,274; and 6,361,945; and US Patent Application Pub. Nos. 20060068417A1 and 20060194240A1).

### EXAMPLES

The following examples are provided to illustrate certain disclosed embodiments and are not to be construed as limiting the scope of this disclosure in any way.

*General Reagents and Methods.* Unless otherwise indicated, amplifications were performed using a Panther Fusion instrument with Open Access functionality (Hologic, Inc., San Diego, CA). Material to be used as amplification targets or controls were diluted in suitable media, e.g., Solution Transport media (Hologic Inc. Cat. No. 101768) or Simulated negative matrix (liquid Amies Tarnsport medium from Copan Flock, Cat. No. 499C.RD combined with 0.03% mucin and 1E4 HeLa cells/mL). Nucleic acid was extracted from isolates using a non-specific target capture procedure as described in US Patent App. Pub. 2013/0209992.

PCR reactions were typically assembled as follows:
Panther Fusion Open Access 12 well cartridge (each well contains a lyophilized single dose unit made of EDTA, dNTPs, Trehalose, Go Taq^{®} MDx Hot Start Polymerase, buffer containinf Tris and non-acetylated BSA, GoScript Reverse Transcriptase and RNasin Plus)
20µL of Primer Probe Reagent (containing primers, probe, MgCl₂ and KCl)
5 µL of extracted nucleic acid (target in suitable diluent)
=25 µL total reaction volume.

The oligomer primer and probe combinations shown in Table 2 were tested in various amplification and detection experiments. Oligomers primers and probes are referred to by their SEQ ID NO (see Table 10 below for sequence disclosure).

**Table 2**

| Mix | Oligomer 1 (Forward primer) SEQ ID NO | Oligomer 3 (Probe) SEQ ID NO | Oligomer 2 (Reverse primer) SEQ ID NO(s) |
|---|---|---|---|
| 1 | 1 | 2 | 3 |
| 2 | 4 | 5 | 6 |
| 3 | 7 | 8 | 9 |
| 4 | 10 | 11 | 12 |
| 5 | 13 | 14 | 15 |
| 5.1 | 16 | 25 | 17 |
| 5.2 | 16 | 26 | 17 |
| 5.3 | 16 | 18 | 17 |
| 6 | 19 | 21 | 20 |
| 7 | 22 | 24 | 23 |
| 8.1 | 27 | 29 | 28 |
| 8.2 | 30 | 29 | 28 |
| 9 | 31 | 33 | 32 |
| 10 | 34 | 36 | 35 |
| 11 | 37 | 39 | 38 |

Oligomers with the sequences of the following SEQ ID NOs contained 5-methyl-dC (5-me-dC) residues at the positions indicated below. SEQ ID NO: 4: 5-me-dC at positions 8, 18, and 24. SEQ ID NO: 5: 5-me-dC at positions 2, 4, 15, 19, and 21. SEQ ID NO: 6: 5-me-dC at positions 7, 8, 11, and 13. SEQ ID NO: 16: 5-me-dC at positions 7, 8, 10, and 21. SEQ ID NO: 17: 5-me-dC at positions 6, 7, 9, 11, 12, 15, and 19. SEQ ID NO: 18: 5-me-dC at positions 17 and 23. SEQ ID NO: 23: 5-me-dC at positions 5, 7, 11, 13, and 19. SEQ ID NO: 25: 5-me-dC at positions 17, 23, 25, 26, and 28. SEQ ID NO: 26: 5-me-dC at positions 8, 14, 15, 16, 17, and 28. SEQ ID NOs: 8 and 11 contained an inosine at the sequence characters represented by "N".

### Example 1. Mixes 1-5.

The following samples were tested with a series of primer and probe mixes: Strep A lysate at 1000, 100, and 50 CFU/mL; Strep A cell suspension at 1000, 100, and 50 CFU/mL. The primer and probe mixes used were mixes 1, 2, 3, 4, and 5 shown in Table 2. Detection results are shown in Table 3 below.

**Table 3**

| **Mix** | **Sample Type** | **Concentration (CFU/mL)** | **Mean RFU** | **Std Dev RFU** | **Mean Ct** | **Std Dev Ct** |
|---|---|---|---|---|---|---|
| 1 | Cell Suspension | 1000 | 28842.17 | 1048.98 | 32.38 | 0.15 |
| | | 100 | 14866.96 | 2432.87 | 38.19 | 0.72 |
| | | 50 | 15806.98 | 2263.50 | 37.71 | 0.95 |
| | Lysate | 1000 | 26974.38 | 2913.34 | 32.07 | 0.25 |
| | | 100 | 21993.75 | 1866.30 | 35.59 | 0.95 |
| | | 50 | 17249.00 | 6272.70 | 37.14 | 1.62 |
| 2 | Cell Suspension | 1000 | 26820.05 | 3482.99 | 33.35 | 0.19 |
| | | 100 | 6501.94 | 3220.47 | 38.35 | 1.47 |
| | | 50 | 9860.18 | 5602.93 | 37.69 | 1.39 |
| | Lysate | 1000 | 25316.21 | 1501.96 | 31.91 | 0.46 |
| | | 100 | 11752.89 | 2698.65 | 37.27 | 0.46 |
| | | 50 | 10511.86 | 2030.89 | 37.16 | 0.80 |
| 3 | Cell Suspension | 1000 | 14680.33 | 363.35 | 33.82 | 0.57 |
| | | 100 | 12219.53 | 1256.98 | 35.93 | 2.34 |
| | | 50 | 3081.12 | 7165.40 | 37.74 | NA |
| | Lysate | 1000 | 14088.73 | 530.86 | 32.82 | 0.21 |
| | | 100 | 12250.64 | 1153.24 | 36.45 | 1.51 |
| | | 50 | 12417.11 | 124.03 | 36.72 | 0.35 |
| 4 | Cell Suspension | 1000 | 17729.73 | 1984.07 | 33.55 | 1.21 |
| | | 100 | 13168.15 | 1296.70 | 37.52 | 0.52 |
| | | 50 | 4759.33 | 7249.70 | 38.54 | NA |
| | Lysate | 1000 | 17925.04 | 962.64 | 32.73 | 0.62 |
| | | 100 | 15400.43 | 2173.18 | 35.57 | 0.65 |
| | | 50 | 15447.76 | 1563.11 | 33.17 | 6.20 |
| 5 | Cell Suspension | 1000 | 852.81 | 381.66 | 23.52 | NA |
| | | 100 | 104.54 | 1227.62 | NA | NA |
| | | 50 | 1107.60 | 217.90 | 37.35 | 10.46 |
| | Lysate | 1000 | 688.75 | 1091.78 | 26.30 | 4.61 |
| | | 100 | -979.01 | 321.18 | NA | NA |
| | | 50 | 527.13 | 1330.39 | 22.81 | NA |

After amplification, mix 1, 2, 3, and 4 gave signal (RFU) over the threshold. Mix 5 did not give signal over the threshold. Mix 1, 2, 3 and 4 showed comparable Ct values for all target concentrations of cell suspension or lysate, but Mix 3 and 4 showed relatively increased variability at low target concentrations (50 CFU/mL). In addition, Mix 3 and 4 showed significantly decreased RFU values compared to Mix 1 and 2 for 1000 CFU/mL.

### Example 2. Mixes 2, 5.3, 6, and 7.

The following samples were tested with a series of primer and probe mixes: Strep A strain NCTC 12696 lysate at 50 and 150 CFU/mL; Strep G lysate at 150 CFU/mL; and a second Strep A strain NCTC 8709 cell suspension at 500 CFU. The primer and probe mixes used were mixes 2, 5.3, 6, and 7. Detection results are shown in Table 4 below.

**Table 4**

| **Mix** | **Sample Type** | **Target** | **Concentration (CFU/mL)** | **Mean RFU** | **Std Dev RFU** | **Mean Ct** | **Std Dev Ct** |
|---|---|---|---|---|---|---|---|
| 2 | Cell suspension | Strep A NCTC 8709 | 500 | 7103.98 | 5795.54 | 39.55 | 1.20 |
| | Lysate | Strep A NCTC 12696 | 150 | 21680.69 | 1491.80 | 35.74 | 0.28 |
| | | | 50 | 18010.94 | 5040.74 | 37.42 | 0.72 |
| | | Strep G | 150 | 437.08 | 68.40 | NA | NA |
| | Negative Ctrl | None | 0 | 380.8 | 21.01 | NA | NA |
| 5.3 | Cell suspension | Strep A NCTC 8709 | 500 | 13881.80 | 2615.35 | 38.91 | 0.83 |
| | Lysate | Strep A NCTC 12696 | 150 | 16157.65 | 1101.18 | 36.71 | 0.60 |
| | | | 50 | 15396.74 | 1571.79 | 37.86 | 0.98 |
| | | Strep G | 150 | 848.77 | 223.62 | NA | NA |
| | Negative Ctrl | None | 0 | -555.25 | 1019.25 | NA | NA |
| 6 | Cell suspension | Strep A NCTC 8709 | 500 | 6698.88 | 1671.61 | 38.40 | 1.11 |
| | Lysate | Strep A NCTC 12696 | 150 | 16870.67 | 433.86 | 34.95 | 0.32 |
| | | | 50 | 12679.59 | 3063.03 | 35.24 | 0.87 |
| | | Strep G | 150 | 8556.71 | 1464.36 | 36.79 | 0.43 |
| | Negative Ctrl | None | 0 | 1524.79 | 413.82 | NA | NA |
| 7 | Cell suspension | Strep A NCTC 8709 | 500 | 9914.05 | 278.47 | 37.14 | 1.74 |
| | Lysate | Strep A NCTC 12696 | 150 | 13562.86 | 433.81 | 35.56 | 0.80 |
| | | | 50 | 10027.46 | 1169.11 | 38.36 | 0.64 |
| | | Strep G | 150 | 8214.99 | 1485.13 | 37.70 | 0.82 |
| | Negative Ctrl | None | 0 | 8138.40 | 1469.94 | 37.75 | 0.55 |

Strep G used herein was *Streptococcus dysgalactiae* (available from American Type Culture Collection (ATCC), Manassas, VA, Cat. No. ATCC-12394), which is not a GAS but is nonetheless genetically closely related to GAS.

Mixes 6 and 7 showed cross-reaction with the Strep G sample and mix 7 also gave signal above the threshold in the negative control. Mixes 2 and 5.3 specifically detected Strep A and mix 5.3 detected strain NCTC 8709 with the highest signal strength.

### Example 3. Mixes 5.1, 5.2, and 5.3.

The following samples were tested with a series of primer and probe mixes: Strep A strain NCTC 12696 lysate at 50, 150 and 500 CFU/mL. The primer and probe mixes used were mixes 5.1, 5.2, and 5.3.

Detection results are shown in Table 5 below.

**Table 5**

| **Mix** | **Sample Type** | **Target** | **Concentration (CFU/mL)** | **Mean RFU** | **Std Dev RFU** | **Mean Ct** | **Std Dev Ct** |
|---|---|---|---|---|---|---|---|
| 5.1 | Lysate | Strep A NCTC 12696 | 500 | 16813.40 | 580.93 | 33.83 | 0.05 |
| | | | 150 | 12751.78 | 420.31 | 35.92 | 0.22 |
| | | | 50 | 10656.34 | 721.63 | 37.43 | 0.11 |
| | Negative Ctrl | Negative | 0 | 47.63 | 835.69 | NA | NA |
| 5.2 | Lysate | Strep A NCTC 12696 | 500 | 18999.21 | 1102.20 | 29.57 | 5.17 |
| | | | 150 | 16986.82 | 542.58 | 34.02 | 0.95 |
| | | | 50 | 14982.06 | 2453.89 | 36.74 | 1.09 |
| | Negative Ctrl | Negative | 0 | -1518.39 | 107.48 | NA | NA |
| 5.3 | Lysate | Strep A NCTC 12696 | 500 | 10890.63 | 2197.03 | 35.51 | 0.52 |
| | | | 150 | 9595.35 | 942.43 | 37.21 | 0.56 |
| | | | 50 | 7838.01 | 1656.43 | 38.51 | 0.97 |
| | Negative Ctrl | Negative | 0 | -492.15 | 851.79 | NA | NA |

After amplification, mix 5.2 gave the highest signal over threshold.

A panel of Streptococcus species as shown in Table 6 was tested with mixes 5.1, 5.2, and 5.3. Mix 5.1 showed cross reactivity with the sample containing *Streptococcus canis* and *Streptococcus constellates.* Mixes 5.2 and 5.3 were negative with all samples except GAS.

**Table 6**

| **Mix** | **Sample Type** | **Target** | **Concentration (CFU/mL)** | **Mean RFU** | **Std Dev RFU** | **Mean Ct** | **Std Dev Ct** |
|---|---|---|---|---|---|---|---|
| 5.1 | Negative Ctrl | None | 0 | -116.35 | 106.93 | NA | NA |
| | Positive Ctrl | Strep A NCTC 12696 | 150 | 15433.56 | 1403.81 | 38.03 | 0.29 |
| | Lysate | Streptococcus agalactiae | 1E6 | 4.09 | 106.20 | NA | NA |
| | | Streptococcus mitis | 1E6 | | | | |
| | Lysate | Streptococcus bovis | 1E6 | -57.35 | 90.93 | NA | NA |
| | | Streptococcus mutans | 1E6 | | | | |
| | Lysate | Streptococcus canis | 1E6 | 3102.47 | 83.75 | 43.24 | 0.10 |
| | | Streptococcus constellatus | 1E6 | | | | |
| | Lysate | Streptococcus gordonii | 1E6 | 53.63 | 141.49 | NA | NA |
| | | Streptococcus intermedius | 1E6 | | | | |
| | Lysate | Streptococcus oralis | 1E6 | 20.75 | 104.51 | NA | NA |
| | | Streptococcus suis | 1E6 | | | | |
| | Cell suspension | Streptococcus pneumoniae | 1E6 | -162.92 | 233.56 | NA | NA |
| | | Streptococcus salivarius | 1E6 | | | | |
| | Lysate | Streptococcus sanguinis | 1E6 | -6.51 | 120.42 | NA | NA |
| | | Streptococcus anginosus | 1E6 | | | | |
| | Lysate | StrepC | 150 | -202.31 | 100.60 | NA | NA |
| | Lysate | StrepG | 150 | -74.21 | 57.38 | NA | NA |
| 5.2 | Negative Ctrl | None | 0 | -81.06 | 154.49 | NA | NA |
| | Positive Ctrl | Strep A NCTC 12696 | 150 | 16432.37 | 630.69 | 37.89 | 0.49 |
| | Lysate | Streptococcus agalactiae | 1E6 | 193.03 | 33.84 | NA | NA |
| | | Streptococcus mitis | 1E6 | | | | |
| | Lysate | Streptococcus bovis | 1E6 | 16.55 | 172.48 | NA | NA |
| | | Streptococcus mutans | 1E6 | | | | |
| | Lysate | Streptococcus canis | 1E6 | 428.29 | 70.06 | NA | NA |
| | | Streptococcus constellatus | 1E6 | | | | |
| | Lysate | Streptococcus gordonii | 1E6 | 154.33 | 44.51 | NA | NA |
| | | Streptococcus intermedius | 1E6 | | | | |
| | Lysate | Streptococcus oralis | 1E6 | 3.62 | 271.79 | NA | NA |
| | | Streptococcus suis | 1E6 | | | | |
| | Cell suspension | Streptococcus pneumoniae | 1E6 | 9.18 | 153.57 | NA | NA |
| | | Streptococcus salivarius | 1E6 | | | | |
| | Lysate | Streptococcus sanguinis | 1E6 | -35.33 | 100.36 | NA | NA |
| | | Streptococcus anginosus | 1E6 | | | | |
| | Lysate | StrepC | 150 | -277.30 | 480.02 | NA | NA |
| | Lysate | StrepG | 150 | -24.30 | 193.12 | NA | NA |
| 5.3 | Negative Ctrl | None | 0 | 120.40 | 40.40 | NA | NA |
| | Positive Ctrl | Strep A NCTC 12696 | 150 | 11793.34 | 1924.86 | 38.78 | 0.62 |
| | Lysate | Streptococcus agalactiae | 1E6 | 27.89 | 84.90 | NA | NA |
| | | Streptococcus mitis | 1E6 | | | | |
| | Lysate | Streptococcus bovis | 1E6 | 86.62 | 11.60 | NA | NA |
| | | Streptococcus mutans | 1E6 | | | | |
| | Lysate | Streptococcus canis | 1E6 | 139.46 | 66.31 | NA | NA |
| | | Streptococcus constellatus | 1E6 | | | | |
| | Lysate | Streptococcus gordonii | 1E6 | 3.21 | 93.23 | NA | NA |
| | | Streptococcus intermedius | 1E6 | | | | |
| | Lysate | Streptococcus oralis | 1E6 | -36.25 | 94.70 | NA | NA |
| | | Streptococcus suis | 1E6 | | | | |
| | Cell suspension | Streptococcus pneumoniae | 1E6 | 11.54 | 130.94 | NA | NA |
| | | Streptococcus salivarius | 1E6 | | | | |
| | Lysate | Streptococcus sanguinis | 1E6 | -14.54 | 68.41 | NA | NA |
| | | Streptococcus anginosus | 1E6 | | | | |
| | Lysate | StrepC | 150 | 22.58 | 91.77 | NA | NA |
| | Lysate | StrepG | 150 | 12.81 | 67.76 | NA | NA |

The Streptococcus species listed in the above table were obtained from ATCC and cultured to provide high titers for testing.

### Example 4. Further characterization of mix 5.2.

Clinical samples (n=36), including samples that had tested positive or negative for Group A Strep in culture, were tested with primer and probe mix 5.2. Detection results are shown in Table 7 below.

**Table 7**

| **Mix** | **Clinical sample ID** | **Culture result** | **GAS RFU** | **GAS Ct** | **GAS Result** | **Agreement** |
|---|---|---|---|---|---|---|
| 5.2 | DLS15-14945 | Negative | 3305.05 | 36.28 | Positive | Discordant |
| | DLS15-14946 | Negative | 918.89 | NA | Negative | Concordant |
| | DLS15-14947 | Negative | 194.09 | NA | Negative | Concordant |
| | DLS15-14948 | Negative | 7793.99 | 40.01 | Positive | Discordant |
| | DLS15-14949 | Negative | 7847.03 | 36.59 | Positive | Discordant |
| | DLS15-14950 | Negative | 3027.99 | 37.28 | Positive | Discordant |
| | DLS15-14951 | Negative | 3207.98 | 36.49 | Positive | Discordant |
| | DLS15-14955 | Negative | 21002.17 | 26.38 | Positive | Discordant |
| | DLS17-048989 | Positive | 19194.49 | 29.38 | Positive | Concordant |
| | DLS17-048990 | Positive | 23148.30 | 12.73 | Positive | Concordant |
| | DLS17-048991 | Positive | 22742.90 | 16.75 | Positive | Concordant |
| | DLS17-048992 | Positive | 24525.45 | 13.93 | Positive | Concordant |
| | DLS17-049047 | Positive | 22444.05 | 28.37 | Positive | Concordant |
| | DLS17-049048 | Positive | 25001.96 | 16.89 | Positive | Concordant |
| | DLS17-049049 | Positive | 27058.36 | 14.96 | Positive | Concordant |
| | DLS17-049050 | Positive | 24112.97 | 14.08 | Positive | Concordant |
| | DLS17-049051 | Positive | 21224.19 | 30.14 | Positive | Concordant |
| | DLS17-049052 | Positive | 27776.66 | 13.67 | Positive | Concordant |
| | DLS17-049053 | Positive | 24721.08 | 13.27 | Positive | Concordant |
| | DLS17-049054 | Positive | 24361.72 | 15.53 | Positive | Concordant |
| | DLS17-049055 | Positive | 21367.32 | 22.69 | Positive | Concordant |
| | DLS17-049071 | Positive | 23966.89 | 20.41 | Positive | Concordant |
| | DLS17-049072 | Positive | 17016.34 | 37.40 | Positive | Concordant |
| | DLS17-049073 | Positive | 22517.66 | 27.74 | Positive | Concordant |
| | DLS17-049074 | Positive | 23823.43 | 21.64 | Positive | Concordant |
| | DLS17-049087 | Positive | 24592.59 | 21.14 | Positive | Concordant |
| | DLS17-049088 | Positive | 18266.45 | 25.10 | Positive | Concordant |
| | DLS17-049089 | Positive | 27103.44 | 15.41 | Positive | Concordant |
| | DLS17-049090 | Positive | 24210.23 | 15.91 | Positive | Concordant |
| | DLS17-049091 | Positive | 22700.32 | 15.10 | Positive | Concordant |
| | DLS17-049092 | Positive | 26514.28 | 14.65 | Positive | Concordant |
| | DLS17-049365 | Positive | 24915.95 | 24.35 | Positive | Concordant |
| | DLS17-049366 | Positive | 21586.70 | 14.24 | Positive | Concordant |
| | KH18-01040 | Negative | 125.73 | NA | Negative | Concordant |
| | KH18-01041 | Negative | 13136.07 | 38.68 | Positive | Discordant |
| | KH18-01042 | Negative | 192.56 | NA | Negative | Concordant |
| | Negative Control | NA | -64.38 | NA | Negative | NA |
| | Positive Control | NA | 18818.97 | 35.75 | Positive | NA |

Clinical samples listed in the table above were obtained from Discovery Life Sciences.

After amplification, 7 samples that were negative for Group A Strep by culture tested positive using mix 5.2 primer and probe set. These results are considered false positives at this point. All cultures that tested positive for Strep A also tested positive using the mix 5.2 primer and probe set.

### Example 5. Mixes 5.2, 8.1, 8.2, 9, 10, and 11.

Mixes 8, 9, 10 and 11 were tested for potential cross reactivity with a panel of Streptococcus species. Lysates or cell suspensions of Streptococcus species were tested in triplicate at 1 x 10⁶ CFU/mL and included: *S*. *agalactiae*, *S. mitis*, *S bovis*, *S. mutans, S. sanguinis*, *S.pnemoniae* (cs), *S*. *salivarius* (cs), *S*. *anginosus*, *S. cani*, *S. constellatus*, *S. gordoonii*, *S. intermedins*, *S. oralis*, *S. suis*, Group C Strep, and Group G Strep. Organisms for which cell suspensions were used are indicated with (cs); all other samples were lysates. In addition, a separate set of samples of these organisms was spiked with 2,000 CPU of Strep A.

Detection results are shown in Table 8 below.

**Table 8**

| **Mix** | **Target** | **GAS target** | **Mean RFU** | **Std Dev RFU** | **Mean Ct** | **Std Dev Ct** |
|---|---|---|---|---|---|---|
| 5.2 | S. canis | Unspiked | 1515.38 | 206.73 | NA | NA |
| | S. agalactiae | Unspiked | 46.24 | 264.54 | NA | NA |
| | S. anginosus & S. constellatus | Unspiked | -116.15 | 20.88 | NA | NA |
| | S. gordonii & S. intermedius | Unspiked | -91.96 | 258.97 | NA | NA |
| | S. mitis & S. bovis | Unspiked | -53.93 | 160.15 | NA | NA |
| | S. mutans & S. sanguinis | Unspiked | -81.54 | 184.67 | NA | NA |
| | S. oralis & S. suis | Unspiked | -51.51 | 177.91 | NA | NA |
| | S. pneumoniae & S. salivarius | Unspiked | -197.42 | 83.57 | NA | NA |
| 8.1 | S. canis | Unspiked | 106.81 | 315.09 | NA | NA |
| | S. agalactiae | Unspiked | 198.92 | 52.34 | NA | NA |
| | S. anginosus & S. constellatus | Unspiked | -68.08 | 243.20 | NA | NA |
| | S. gordonii & S. intermedius | Unspiked | -312.67 | 212.95 | NA | NA |
| | S. mitis & S. bovis | Unspiked | 160.76 | 346.72 | NA | NA |
| | S. mutans & S. sanguinis | Unspiked | 292.16 | 50.61 | NA | NA |
| | S. oralis & S. suis | Unspiked | -318.06 | 77.84 | NA | NA |
| | S. pneumoniae & S. salivarius | Unspiked | 109.49 | 308.66 | NA | NA |
| 8.2 | S. canis | Unspiked | 99.67 | 273.71 | NA | NA |
| | S. agalactiae | Unspiked | -92.15 | 217.60 | NA | NA |
| | S. anginosus & S. constellatus | Unspiked | -253.43 | 127.00 | NA | NA |
| | S. gordonii & S. intermedius | Unspiked | 82.37 | 217.62 | NA | NA |
| | S. mitis & S. bovis | Unspiked | 70.76 | 312.25 | NA | NA |
| | S. mutans & S. sanguinis | Unspiked | -233.61 | 23.67 | NA | NA |
| | S. oralis & S. suis | Unspiked | -64.99 | 212.45 | NA | NA |
| | S. pneumoniae & S. salivarius | Unspiked | -93.60 | 239.08 | NA | NA |
| 9 | S. canis | Unspiked | -223.67 | 458.30 | NA | NA |
| | S. agalactiae | Unspiked | 32.81 | 366.50 | NA | NA |
| | S. anginosus & S. constellatus | Unspiked | 134.83 | 308.75 | NA | NA |
| | S. gordonii & S. intermedius | Unspiked | 339.49 | 612.70 | NA | NA |
| | S. mitis & S. bovis | Unspiked | 29.53 | 220.52 | NA | NA |
| | S. mutans & S. sanguinis | Unspiked | 85.70 | 322.28 | NA | NA |
| | S. oralis & S. suis | Unspiked | 52.38 | 312.06 | NA | NA |
| | S. pneumoniae & S. salivarius | Unspiked | -45.84 | 310.65 | NA | NA |
| 10 | S. canis | Unspiked | -25.07 | 120.23 | NA | NA |
| | S. agalactiae | Unspiked | -69.47 | 128.68 | NA | NA |
| | S. anginosus & S. constellatus | Unspiked | 51.04 | 224.58 | NA | NA |
| | S. gordonii & S. intermedius | Unspiked | -239.53 | 462.17 | NA | NA |
| | S. mitis & S. bovis | Unspiked | -114.05 | 24.72 | NA | NA |
| | S. mutans & S. sanguinis | Unspiked | 141.49 | 16.82 | NA | NA |
| | S. oralis & S. suis | Unspiked | 37.12 | 104.39 | NA | NA |
| | S. pneumoniae & S. salivarius | Unspiked | 5.49 | 156.46 | NA | NA |
| 11 | S. canis | Unspiked | -289.98 | 86.01 | NA | NA |
| | S. agalactiae | Unspiked | 57.62 | 279.46 | NA | NA |
| | S. anginosus & S. constellatus | Unspiked | -336.74 | 128.32 | NA | NA |
| | S. gordonii & S. intermedius | Unspiked | 43.35 | 547.70 | NA | NA |
| | S. mitis & S. bovis | Unspiked | -242.30 | 88.74 | NA | NA |
| | S. mutans & S. sanguinis | Unspiked | -53.60 | 276.62 | NA | NA |
| | S. oralis & S. suis | Unspiked | -215.59 | 24.82 | NA | NA |
| | S. pneumoniae & S. salivarius | Unspiked | -262.92 | 520.42 | NA | NA |
| 5.2 | S. canis | Spiked | 18573.91 | 2263.68 | 35.06 | 0.42 |
| | S. agalactiae | Spiked | 8672.10 | 1107.50 | 35.91 | 0.27 |
| | S. anginosus & S. constellatus | Spiked | 20313.76 | 1103.63 | 35.20 | 0.21 |
| | S. gordonii & S. intermedius | Spiked | 20626.12 | 1974.08 | 34.89 | 0.23 |
| | S. mitis & S. bovis | Spiked | 18590.73 | 635.40 | 36.20 | 0.09 |
| | S. mutans & S. sanguinis | Spiked | 18471.04 | 731.54 | 35.83 | 0.15 |
| | S. oralis & S. suis | Spiked | 21620.52 | 378.78 | 35.20 | 0.18 |
| | S. pneumoniae & S. salivarius | Spiked | 16689.04 | 1240.91 | 35.77 | 0.10 |
| 8.1 | S. canis | Spiked | 16922.65 | 1652.15 | 34.85 | 0.36 |
| | S. agalactiae | Spiked | 7460.93 | 2909.84 | 35.11 | 0.30 |
| | S. anginosus & S. constellatus | Spiked | 15096.55 | 1973.78 | 35.33 | 0.34 |
| | S. gordonii & S. intermedius | Spiked | 15620.29 | 3213.41 | 34.33 | 0.22 |
| | S. mitis & S. bovis | Spiked | 13767.15 | 1410.91 | 35.92 | 0.10 |
| | S. mutans & S. sanguinis | Spiked | 16793.09 | 1241.40 | 35.45 | 0.13 |
| | S. oralis & S. suis | Spiked | 13143.24 | 810.46 | 35.36 | 0.03 |
| | S. pneumoniae & S. salivarius | Spiked | 18333.44 | 299.22 | 35.18 | 0.12 |
| 8.2 | S. canis | Spiked | 18884.28 | 337.65 | 35.28 | 0.25 |
| | S. agalactiae | Spiked | 12651.00 | 2502.61 | 35.50 | 0.20 |
| | S. anginosus & S. constellatus | Spiked | 15518.60 | 2377.28 | 35.31 | 0.13 |
| | S. gordonii & S. intermedius | Spiked | 16765.18 | 1358.45 | 35.04 | 0.06 |
| | S. mitis & S. bovis | Spiked | 15736.02 | 424.04 | 35.87 | 0.33 |
| | S. mutans & S. sanguinis | Spiked | 16584.07 | 423.56 | 35.63 | 0.09 |
| | S. oralis & S. suis | Spiked | 13495.45 | 2142.89 | 35.34 | 0.12 |
| | S. pneumoniae & S. salivarius | Spiked | 17685.48 | 110.74 | 35.06 | 0.02 |
| 9 | S. canis | Spiked | -401.00 | 123.08 | NA | NA |
| | S. agalactiae | Spiked | 13975.83 | 1903.19 | 37.23 | 0.99 |
| | S. anginosus & S. constellatus | Spiked | 14800.09 | 899.23 | 37.04 | 0.68 |
| | S. gordonii & S. intermedius | Spiked | 13925.30 | 1355.99 | 37.35 | 0.72 |
| | S. mitis & S. bovis | Spiked | 14073.63 | 2748.45 | 37.76 | 0.83 |
| | S. mutans & S. sanguinis | Spiked | 12932.07 | 1349.80 | 37.93 | 0.55 |
| | S. oralis & S. suis | Spiked | 12860.14 | 4304.29 | 37.66 | 0.84 |
| | S. pneumoniae & S. salivarius | Spiked | 14300.43 | 917.21 | 37.39 | 0.29 |
| 10 | S. canis | Spiked | 42.44 | 112.55 | NA | NA |
| | S. agalactiae | Spiked | 1087.81 | 652.81 | NA | NA |
| | S. anginosus & S. constellatus | Spiked | 1750.11 | 952.32 | 39.98 | NA |
| | S. gordonii & S. intermedius | Spiked | 669.59 | 264.46 | NA | NA |
| | S. mitis & S. bovis | Spiked | 1119.50 | 870.67 | 44.50 | NA |
| | S. mutans & S. sanguinis | Spiked | 1035.68 | 783.10 | NA | NA |
| | S. oralis & S. suis | Spiked | 1655.49 | 1035.61 | 40.28 | NA |
| | S. pneumoniae & S. salivarius | Spiked | 1017.11 | 785.95 | NA | NA |
| 11 | S. canis | Spiked | 24391.76 | 854.45 | 36.59 | 0.89 |
| | S. agalactiae | Spiked | 22016.62 | 988.85 | 35.99 | 0.11 |
| | S. anginosus & S. constellatus | Spiked | 23504.57 | 1786.36 | 36.49 | 0.52 |
| | S. gordonii & S. intermedius | Spiked | 20149.34 | 1327.60 | 35.83 | 0.39 |
| | S. mitis & S. bovis | Spiked | 24265.78 | 2328.58 | 35.85 | 0.49 |
| | S. mutans & S. sanguinis | Spiked | 25354.94 | 998.95 | 36.76 | 0.73 |
| | S. oralis & S. suis | Spiked | 23461.68 | 2043.33 | 36.16 | 0.32 |
| | S. pneumoniae & S. salivarius | Spiked | 22581.58 | 2763.00 | 36.92 | 0.96 |

Clinical samples listed in the table above were obtained from Discovery Life Sciences.

The Streptococcus species listed in the above table were obtained from ATCC and cultured to provide high titers for testing.

The results for mixes 5.2, 8.1, 8.2, 9, 10, and 11 no signal above the threshold was detected for the unspiked samples, indicating lack of cross-reactivity. Mixes 5.2, 8.1, 8.2, 9, and 11 gave signals above the threshold in the spiked samples.

### Example 6. Further characterisation of mixes 5.2, 8.2, and 11.

Throat swabs in Amies medium (n=36), of which 25 samples previously tested positive and 11 samples previously tested negative for Group A Strep in culture, were tested with mixes 5.2, 8.2, and 11. Detection results are shown in Table 9 below.

**Table 9**

| **Sample ID** | **Culture Result** | **GAS Mix 5.2** | **GAS Mix 8.2** | **GAS Mix 11** |
|---|---|---|---|---|
| DLS15-14945 | Negative | Positive | Negative | Negative |
| DLS15-14946 | Negative | Negative | Negative | Negative |
| DLS15-14947 | Negative | Negative | Positive | Negative |
| DLS15-14948 | Negative | Positive | Negative | Negative |
| DLS15-14949 | Negative | Positive | Negative | Negative |
| DLS15-14950 | Negative | Positive | Negative | Negative |
| DLS15-14951 | Negative | Positive | Negative | Negative |
| DLS15-14955 | Negative | Positive | Positive | Positive |
| DLS17-048989 | Positive | Positive | Positive | Positive |
| DLS17-048990 | Positive | Positive | Positive | Positive |
| DLS17-048991 | Positive | Positive | Positive | Positive |
| DLS17-048992 | Positive | Positive | Positive | Positive |
| DLS17-049047 | Positive | Positive | Positive | Positive |
| DLS17-049048 | Positive | Positive | Positive | Positive |
| DLS17-049049 | Positive | Positive | Positive | Positive |
| DLS17-049050 | Positive | Positive | Positive | Positive |
| DLS17-049051 | Positive | Positive | Positive | Positive |
| DLS17-049052 | Positive | Positive | Positive | Positive |
| DLS17-049053 | Positive | Positive | Positive | Positive |
| DLS17-049054 | Positive | Positive | Positive | Positive |
| DLS17-049055 | Positive | Positive | Positive | Positive |
| DLS17-049071 | Positive | Positive | Positive | Positive |
| DLS17-049072 | Positive | Positive | Negative | Negative |
| DLS17-049073 | Positive | Positive | Positive | Positive |
| DLS17-049074 | Positive | Positive | Positive | Positive |
| DLS17-049087 | Positive | Positive | Positive | Positive |
| DLS17-049088 | Positive | Positive | Positive | Positive |
| DLS17-049089 | Positive | Positive | Positive | Positive |
| DLS17-049090 | Positive | Positive | Positive | Positive |
| DLS17-049091 | Positive | Positive | Positive | Positive |
| DLS17-049092 | Positive | Positive | Positive | Positive |
| DLS17-049365 | Positive | Positive | Positive | Positive |
| DLS17-049366 | Positive | Positive | Positive | Positive |
| KH18-01040 | Negative | Negative | Negative | Negative |
| KH18-01041 | Negative | Positive | Negative | Negative |
| KH18-01042 | Negative | Negative | Negative | Negative |

Each mix gave positive results for all or nearly all of the samples that were positive for GAS by culture. Amplification with mix 5.2, 8.2, and 11 resulted in 36%, 82%, and 91% negative agreement with the culture results, respectively.

**Table 10. Disclosed Sequences**

| **[00185]** In the following table, sequences are shown in the 5' to 3' direction. Detection oligomers may be labeled and blocked (rendered nonextendable) as described above. | | |
|---|---|---|
| SEQ ID NO | Description | Sequence† |
| 1 | Forward Amplification Oligomer | GCAAGTAGAACGCTGAGAACTG |
| 2 | Detection Oligomer | CGGTTCAAGGAGTTGCGAACGG |
| 3 | Reverse Amplification Oligomer | TATCCCCCGCTATGAGGTAG |
| 4 | Forward Amplification Oligomer | GGGATAACTATTGGAAACGATAGC |
| 5 | Detection Oligomer | CCGCATAAGAGAGACTAACGCATGTTAG |
| 6 | Reverse Amplification Oligomer | GCAGGTCCATCTCATAGTGGAG |
| 7 | Forward Amplification Oligomer | TTGTAGGACTGCGACGTG |
| 8 | Detection Oligomer | AGAAGAANTACCTGGGAAGGTAAGCC |
| 9 | Reverse Amplification Oligomer | GTACTCAGGATACTGCTAGGGCTCAA |
| 10 | Forward Amplification Oligomer | CAGGATAGGTAGGAGCCATTGACTT |
| 11 | Detection Oligomer | CGAATGAGGCGNTGTTGGGATACTACC |
| 12 | Reverse Amplification Oligomer | TCAGACACTGTCTCCGATAGGGATAAC |
| 13 | Forward Amplification Oligomer | TCAGTAGCGGTAGCTTTGACTGTT |
| 14 | Detection Oligomer | CTTCCCTAGGATTACCATCACCGTTAG |
| 15 | Reverse Amplification Oligomer | TTGCGGGATTGTTTGCTGCAAGATC |
| 16 | Forward Amplification Oligomer | CTAATACCGCATAAGAGAGACTAAC |
| 17 | Reverse Amplification Oligomer | CTTTACCTCACCAACTAGCTAATAC |
| 18 | Detection Oligomer | AGTAATTTAAAAGGGGCAATTGCTC |
| 19 | Forward Amplification Oligomer | CCGAGCGTTGTCCGGATTTATT |
| 20 | Reverse Amplification Oligomer | CTTCTGCACTCAAGTTCTCCAGTTT |
| 21 | Detection Oligomer | CCAAAGCGTACATTGGTTGAGCCAATGCCT |
| 22 | Forward Amplification Oligomer | GTTGAAACTCAAAGGAATTGACGGG |
| 23 | Reverse Amplification Oligomer | AAAACTCTATCTCTAGAGCGGGCAT |
| 24 | Detection Oligomer | GGCCCGCACAAGCGGTGGAGCATGTGGT |
| 25 | Detection Oligomer | AGTAATTTAAAAGGGGCAATTGCTCCAC |
| 26 | Detection Oligomer | AGTGGAGCAATTGCCCCTTTTAAATTACT |
| 27 | Forward Amplification Oligomer | CTGGTGCTTGCACCGGTTC |
| 28 | Reverse Amplification Oligomer | CCTTTTAAATTACTAACATGCGTTAGTCTC |
| 29 | Detection Oligomer | TCTTATGCGGTATTAGCTATCGTTTCCAATAG |
| 30 | Forward Amplification Oligomer | GGTGCTTGCACCGGTTC |
| 31 | Forward Amplification Oligomer | CCTTCTACCTCCACTACACTGA |
| 32 | Reverse Amplification Oligomer | GTCGAGTGTCTGTGTAGATTTCAGCC |
| 33 | Detection Oligomer | CAAACGGTACAAATCATCCAGAGTGTC |
| 34 | Forward Amplification Oligomer | GTCCTCAATCAAACCTTGTCCTACC |
| 35 | Reverse Amplification Oligomer | GGTAACAGAAATCCTTGATGAGTTGCGG |
| 36 | Detection Oligomer | GAGGTAAGAAATAGTAGCGAGTGCGG |
| 37 | Forward Amplification Oligomer | CCAATCAATACCATATTCCTTATCCC |
| 38 | Reverse Amplification Oligomer | CGGCAACCTCTTCAGTGGTTTC |
| 39 | Detection Oligomer | CCTTGCCAGTATTGAGAGCATTATCAGC |

| | | |
|---|---|---|
| † The symbols for representing the nucleotide characters in the sequences are set forth in the tables of WIPO Standard ST.25 (1998), Appendix 2, Tables 1 and 3. | | |

The further aspects of the present disclosure are set forth in the following numbered paragraphs:
1. A composition or kit comprising at least first and second amplification oligomers, wherein:
   (a) the first amplification oligomer and second amplification oligomer are configured to amplify a Group A Streptococcus amplicon;
   (b) the first amplification oligomer:
      (i) comprises a first sequence which is SEQ ID NO: 1, 4, 7, 10, 13, 16, 19, 22, 27, 30, 31, 34, or 37;
      (ii) comprises a sequence with 0, 1, or 2 mismatches to a first sequence which is SEQ ID NO: 1, 4, 7, 10, 13, 16, 19, 22, 27, 30, 31, 34, or 37;
      (iii) competes for hybridization to a Group A Streptococcus (GAS) nucleic acid under stringent conditions with an amplification oligomer whose sequence consists of a first sequence which is SEQ ID NO: 1, 4, 7, 10, 13, 16, 19, 22, 27, 30, 31, 34, or 37; or
      (iv) comprises at least 10, 11, 12, 13, 14, 15, 16, 17, 18, 19, or 20 contiguous nucleotides of a first sequence which is SEQ ID NO: 1, 4, 7, 10, 13, 16, 19, 22, 27, 30, 31, 34, or 37; and
   (c) the second amplification oligomer:
      (i) comprises a second sequence which is SEQ ID NO: 3, 6, 9, 12, 15, 17, 20, 23, 28, 32, 35, or 38;
      (ii) comprises a sequence with 0, 1, or 2 mismatches to a second sequence which is SEQ ID NO: 3, 6, 9, 12, 15, 17, 20, 23, 28, 32, 35, or 38;
      (iii) competes for hybridization to a Group A Streptococcus (GAS) nucleic acid under stringent conditions with an amplification oligomer whose sequence consists of a second sequence which is SEQ ID NO: 3, 6, 9, 12, 15, 17, 20, 23, 28, 32, 35, or 38; or
      (iv) comprises at least 10, 11, 12, 13, 14, 15, 16, 17, 18, 19, or 20 contiguous nucleotides of a second sequence which is SEQ ID NO: 3, 6, 9, 12, 15, 17, 20, 23, 28, 32, 35, or 38.
2. A method of detecting GAS in a sample, the method comprising:
   contacting the sample with at least first and second amplification oligomers, thereby forming a composition,
   performing a nucleic acid amplification reaction in the composition which produces a GAS amplicon in the presence of a GAS nucleic acid, and
   detecting the presence or absence of the at least one amplicon,
   wherein (a) the first amplification oligomer:
      (i) comprises a first sequence which is SEQ ID NO: 1, 4, 7, 10, 13, 16, 19, 22, 27, 30, 31, 34, or 37;
      (ii) comprises a sequence with 0, 1, or 2 mismatches to a first sequence which is SEQ ID NO: 1, 4, 7, 10, 13, 16, 19, 22, 27, 30, 31, 34, or 37;
      (iii) competes for hybridization to a Group A Streptococcus (GAS) nucleic acid under stringent conditions with an amplification oligomer whose sequence consists of a first sequence which is SEQ ID NO: 1, 4, 7, 10, 13, 16, 19, 22, 27, 30, 31, 34, or 37; or
      (iv) comprises at least 10, 11, 12, 13, 14, 15, 16, 17, 18, 19, or 20 contiguous nucleotides of a first sequence which is SEQ ID NO: 1, 4, 7, 10, 13, 16, 19, 22, 27, 30, 31, 34, or 37; and
   (b) the second amplification oligomer:
      (i) comprises a second sequence which is SEQ ID NO: 3, 6, 9, 12, 15, 17, 20, 23, 28, 32, 35, or 38;
      (ii) comprises a sequence with 0, 1, or 2 mismatches to a second sequence which is SEQ ID NO: 3, 6, 9, 12, 15, 17, 20, 23, 28, 32, 35, or 38;
      (iii) competes for hybridization to a Group A Streptococcus (GAS) nucleic acid under stringent conditions with an amplification oligomer whose sequence consists of a second sequence which is SEQ ID NO: 3, 6, 9, 12, 15, 17, 20, 23, 28, 32, 35, or 38; or
      (iv) comprises at least 10, 11, 12, 13, 14, 15, 16, 17, 18, 19, or 20 contiguous nucleotides of a second sequence which is SEQ ID NO: 3, 6, 9, 12, 15, 17, 20, 23, 28, 32, 35, or 38.
3. The composition, kit, or method of any one of the preceding paragraphs,
   wherein the first amplification oligomer competes for hybridization to a Group A Streptococcus (GAS) nucleic acid under stringent conditions with an amplification oligomer whose sequence consists of a first sequence which is SEQ ID NO: 1, 4, 7, 10, 13, 16, 19, 22, 27, 30, 31, 34, or 37.
4. The composition, kit, or method of any one of paragraphs 1-3, wherein the first sequence is the sequence of SEQ ID NO: 1.
5. The composition, kit, or method of any one of paragraphs 1-3, wherein the first sequence is the sequence of SEQ ID NO: 4.
6. The composition, kit, or method of any one of paragraphs 1-3, wherein the first sequence is the sequence of SEQ ID NO: 7.
7. The composition, kit, or method of any one of paragraphs 1-3, wherein the first sequence is the sequence of SEQ ID NO: 10.
8. The composition, kit, or method of any one of paragraphs 1-3, wherein the first sequence is the sequence of SEQ ID NO: 13.
9. The composition, kit, or method of any one of paragraphs 1-3, wherein the first sequence is the sequence of SEQ ID NO: 16.
10. The composition, kit, or method of any one of paragraphs 1-3, wherein the first sequence is the sequence of SEQ ID NO: 19.
11. The composition, kit, or method of any one of paragraphs 1-3, wherein the first sequence is the sequence of SEQ ID NO: 22.
12. The composition, kit, or method of any one of paragraphs 1-3, wherein the first sequence is the sequence of SEQ ID NO: 27.
13. The composition, kit, or method of any one of paragraphs 1-3, wherein the first sequence is the sequence of SEQ ID NO: 30.
14. The composition, kit, or method of any one of paragraphs 1-3, wherein the first sequence is the sequence of SEQ ID NO: 31.
15. The composition, kit, or method of any one of paragraphs 1-3, wherein the first sequence is the sequence of SEQ ID NO: 34.
16. The composition, kit, or method of any one of paragraphs 1-3, wherein the first sequence is the sequence of SEQ ID NO: 37.
17. The composition, kit, or method of any one paragraphs 1-16, wherein the second sequence is the sequence of SEQ ID NO: 3.
18. The composition, kit, or method of any one paragraphs 1-16, wherein the second sequence is the sequence of SEQ ID NO: 6.
19. The composition, kit, or method of any one paragraphs 1-16, wherein the second sequence is the sequence of SEQ ID NO: 9.
20. The composition, kit, or method of any one paragraphs 1-16, wherein the second sequence is the sequence of SEQ ID NO: 12.
21. The composition, kit, or method of any one paragraphs 1-16, wherein the second sequence is the sequence of SEQ ID NO: 15.
22. The composition, kit, or method of any one paragraphs 1-16, wherein the second sequence is the sequence of SEQ ID NO: 17.
23. The composition, kit, or method of any one paragraphs 1-16, wherein the second sequence is the sequence of SEQ ID NO: 20.
24. The composition, kit, or method of any one paragraphs 1-16, wherein the second sequence is the sequence of SEQ ID NO: 23.
25. The composition, kit, or method of any one paragraphs 1-16, wherein the second sequence is the sequence of SEQ ID NO: 28.
26. The composition, kit, or method of any one paragraphs 1-16, wherein the second sequence is the sequence of SEQ ID NO: 32.
27. The composition, kit, or method of any one paragraphs 1-16, wherein the second sequence is the sequence of SEQ ID NO: 35.
28. The composition, kit, or method of any one paragraphs 1-16, wherein the second sequence is the sequence of SEQ ID NO: 38.
29. The composition, kit, or method of any one of the preceding paragraphs,
   wherein the composition or kit further comprises a third oligomer, and the third oligomer:
   (i) comprises a third sequence which is SEQ ID NO: 2, 5, 8, 11, 14, 18, 25, 26, 21, 24, 29, 33, 36, or 39;
   (ii) comprises a sequence with 0, 1, or 2 mismatches to a third sequence which is SEQ ID NO: 2, 5, 8, 11, 14, 18, 25, 26, 21, 24, 29, 33, 36, or 39;
   (iii) competes for hybridization to a Group A Streptococcus (GAS) nucleic acid under stringent conditions with an oligomer whose sequence consists of a third sequence which is SEQ ID NO: 2, 5, 8, 11, 14, 18, 25, 26, 21, 24, 29, 33, 36, or 39; or
   (iv) comprises at least 10, 11, 12, 13, 14, 15, 16, 17, 18, 19, or 20 contiguous nucleotides of a third sequence which is SEQ ID NO: 2, 5, 8, 11, 14, 18, 25, 26, 21, 24, 29, 33, 36, or 39.
30. The composition, kit, or method of paragraph 29, wherein the third sequence is the sequence of SEQ ID NO: 2.
31. The composition, kit, or method of paragraph 29, wherein the third sequence is the sequence of SEQ ID NO: 5.
32. The composition, kit, or method of paragraph 29, wherein the N in SEQ ID NO: 8 is an inosine.
33. The composition, kit, or method of paragraph 29, wherein the N in SEQ ID NO: 11 is an inosine.
34. The composition, kit, or method of any one of paragraphs 29-33, wherein the third oligomer comprises a detectable label.
35. The composition, kit, or method of paragraph 34, wherein the detectable label is fluorescent.
36. The composition, kit, or method of paragraph 35, wherein the third oligomer comprising a fluorescent label further comprises a quencher.
37. The composition, kit, or method of paragraph 35 or 36, wherein at least one oligomer comprising a fluorescent label is a non-extendable oligomer.
38. The composition, kit, or method of any one of the preceding paragraphs,
   wherein at least one oligomer comprises one or more 5-methyl-dC residues.
39. The composition, kit, or method of paragraph 38, wherein the one or more 5-methyl-dC residues comprise residues corresponding to one or more, or all, of positions 8, 18, and 24 of SEQ ID NO: 4, and/or wherein the one or more 5-methyl-dC residues comprise residues corresponding to one or more, or all, of positions 2, 4, 15, 19, and 21 of SEQ ID NO: 5, and/or wherein the one or more 5-methyl-dC residues comprise residues corresponding to one or more, or all, of positions 7, 8, 11, and 13 of SEQ ID NO: 6, and/or wherein the one or more 5-methyl-dC residues comprise residues corresponding to one or more, or all, of positions 7, 8, 10, and 21 of SEQ ID NO: 16, and/or wherein the one or more 5-methyl-dC residues comprise residues corresponding to one or more, or all, of positions 6, 7, 9, 11, 12, 15, and 19 of SEQ ID NO: 18, and/or wherein the one or more 5-methyl-dC residues comprise residues corresponding to one or more, or all, of positions 17 and 23 of SEQ ID NO: 17, and/or wherein the one or more 5-methyl-dC residues comprise residues corresponding to one or more, or all, of positions 5, 7, 11, 13, and 19 of SEQ ID NO: 23, and/or wherein the one or more 5-methyl-dC residues comprise residues corresponding to one or more, or all, of positions 17, 23, 25, 26, and 28 of SEQ ID NO: 25, and/or wherein the one or more 5-methyl-dC residues comprise residues corresponding to one or more, or all, of positions 8, 14, 15, 16, 17, and 28 of SEQ ID NO: 26.
40. The method of any one of paragraphs 2-39, wherein the presence or absence of the amplicon is detected according to the occurrence or non-occurrence of hybridization of the third oligomer to the amplicon.
41. The method of any one of paragraph 2-40, wherein the nucleic acid amplification reaction comprises PCR.
42. The method of any one of paragraphs 2-41, wherein the nucleic acid amplification reaction comprises PCR with a polymerase with 5'-to-3' exonuclease activity, and detecting an amplicon using a probe oligomer comprising a fluorophore and a quencher, wherein exonucleolysis of the probe by the polymerase reduces quenching of fluoresence by the quencher.
43. The composition, kit, or method of any one of the preceding paragraphs,
   wherein the GAS amplicon is about 100-200 nucleotides in length.
44. A kit according to any one of paragraphs 1, 3-39, or 43.
45. A composition according to any one of paragraphs 1, 3-39, or 43.
46. The composition of paragraph 45, which is aqueous, frozen, or lyophilized.
47. Use of a composition or kit of any one of paragraphs 44-46 for detecting or quantifying a GAS nucleic acid in a sample.
48. A method of making an amplification oligonucleotide recited in any one of paragraphs 1-28, comprising the step of chemically synthesizing the amplification oligonucleotide using sequential solid phase oligonucleotide synthesis.
49. A method of making an oligomer recited in any one of paragraphs 29-39, the method comprising chemically synthesizing the oligomer using sequential solid phase oligonucleotide synthesis.

## Claims

1. A composition or kit comprising at least first and second amplification oligomers, wherein:
(a) the first amplification oligomer and second amplification oligomer are configured to amplify a Group A Streptococcus amplicon;
(b) the first amplification oligomer:
(i) comprises a first sequence which is SEQ ID NO: 4;
(ii) comprises a sequence with 0, 1, or 2 mismatches to a first sequence which is SEQ ID NO: 4;
(iii) competes for hybridization to a Group A Streptococcus (GAS) nucleic acid under stringent conditions with an amplification oligomer whose sequence consists of a first sequence which is SEQ ID NO: 4; or
(iv) comprises at least 10, 11, 12, 13, 14, 15, 16, 17, 18, 19, or 20 contiguous nucleotides of a first sequence which is SEQ ID NO: 4; and
(c) the second amplification oligomer:
(i) comprises a second sequence which is SEQ ID NO: 6;
(ii) comprises a sequence with 0, 1, or 2 mismatches to a second sequence which is SEQ ID NO: 6;
(iii) competes for hybridization to a Group A Streptococcus (GAS) nucleic acid under stringent conditions with an amplification oligomer whose sequence consists of a second sequence which is SEQ ID NO: 6; or
(iv) comprises at least 10, 11, 12, 13, 14, 15, 16, 17, 18, 19, or 20 contiguous nucleotides of a second sequence which is SEQ ID NO: 6.

2. A method of detecting GAS in a sample, the method comprising:
contacting the sample with at least first and second amplification oligomers, thereby forming a composition,
performing a nucleic acid amplification reaction in the composition which produces a GAS amplicon in the presence of a GAS nucleic acid, and
detecting the presence or absence of the at least one amplicon,
wherein
(a) the first amplification oligomer:
(i) comprises a first sequence which is SEQ ID NO: 4;
(ii) comprises a sequence with 0, 1, or 2 mismatches to a first sequence which is SEQ ID NO: 4;
(iii) competes for hybridization to a Group A Streptococcus (GAS) nucleic acid under stringent conditions with an amplification oligomer whose sequence consists of a first sequence which is SEQ ID NO: 4; or
(iv) comprises at least 10, 11, 12, 13, 14, 15, 16, 17, 18, 19, or 20 contiguous nucleotides of a first sequence which is SEQ ID NO: 4; and
(b) the second amplification oligomer:
(i) comprises a second sequence which is SEQ ID NO: 6;
(ii) comprises a sequence with 0, 1, or 2 mismatches to a second sequence which is SEQ ID NO: 6;
(iii) competes for hybridization to a Group A Streptococcus (GAS) nucleic acid under stringent conditions with an amplification oligomer whose sequence consists of a second sequence which is SEQ ID NO: 6; or
(iv) comprises at least 10, 11, 12, 13, 14, 15, 16, 17, 18, 19, or 20 contiguous nucleotides of a second sequence which is SEQ ID NO: 6.

3. The composition, kit, or method of claim 1 or claim 2, wherein the first sequence is the sequence of SEQ ID NO: 4.

4. The composition, kit, or method of any one of claims 1-3, wherein the second sequence is the sequence of SEQ ID NO: 6.

5. The composition, kit, or method of any one of the preceding claims, wherein the composition or kit further comprises a third oligomer, and the third oligomer:
(i) comprises a third sequence which is SEQ ID NO: 5;
(ii) comprises a sequence with 0, 1, or 2 mismatches to a third sequence which is SEQ ID NO: 5;
(iii) competes for hybridization to a Group A Streptococcus (GAS) nucleic acid under stringent conditions with an oligomer whose sequence consists of a third sequence which is SEQ ID NO: 5; or
(iv) comprises at least 10, 11, 12, 13, 14, 15, 16, 17, 18, 19, or 20 contiguous nucleotides of a third sequence which is SEQ ID NO: 5.

6. The composition, kit, or method of claim 5, wherein the third sequence is the sequence of SEQ ID NO: 5.

7. The composition, kit, or method of claim 5 or claim 6, wherein the third oligomer comprises a detectable label.

8. The composition, kit, or method of claim 7, wherein the detectable label is fluorescent; preferably,
wherein the third oligomer comprising a fluorescent label further comprises a quencher.

9. The composition, kit, or method of claim 8, wherein at least one oligomer comprising a fluorescent label is a non-extendable oligomer.

10. The composition, kit, or method of any one of the preceding claims, wherein at least one oligomer comprises one or more 5-methyl-dC residues; preferably
wherein the one or more 5-methyl-dC residues comprise residues corresponding to one or more, or all, of positions 2, 4, 15, 19, and 21 of SEQ ID NO: 5.

11. The method of any one of claims 2-10, wherein the presence or absence of the amplicon is detected according to the occurrence or non-occurrence of hybridization of the third oligomer to the amplicon; and/or
wherein the nucleic acid amplification reaction comprises PCR.

12. The method of claim 11, wherein the nucleic acid amplification reaction comprises PCR with a polymerase with 5'-to-3' exonuclease activity, and detecting an amplicon using a probe oligomer comprising a fluorophore and a quencher, wherein exonucleolysis of the probe by the polymerase reduces quenching of fluorescence by the quencher.

13. The composition of any one of claims 1 and 3-10, which is aqueous or lyophilized.

14. Use of a composition or kit of any one of claims 1, 3-10, or 13 for detecting or quantifying a GAS nucleic acid in a sample.

15. A method of making an amplification oligonucleotide recited in any one of claims 1-4 or an oligomer recited in any one of claims 5-10, comprising the step of chemically synthesizing the amplification oligonucleotide using sequential solid phase oligonucleotide synthesis.
